# EUROPEAN PATENT SPECIFICATION

(11) **EP 3 377 619 B1**
(45) Date of publication and mention of the grant of the patent: **17.02.2021**
(21) Application number: 16802001.4
(22) Date of filing: 17.11.2016
(51) Int. Cl.: C12N 9/02, C02F 3/00

(54) **IMPROVED MEANS FOR WATER PURIFICATION**
VERBESSERTE MITTEL ZUR WASSERREINIGUNG
MOYEN AMÉLIORÉ POUR LA PURIFICATION D'EAU

(30) Priority: 20.11.2015 SE 1530178; 25.11.2015 SE 1530180
(43) Date of publication of application: 26.09.2018
(73) Proprietor: Ragn-Sells Recycling AB, 191 29 Sollentuna (SE)
(72) Inventor: LARSSON, Gen, 106 91 Stockholm (SE)
(74) Representative: Herbjørnsen, Rut
(86) International application number: PCT/EP2016/078065
(87) International publication number: WO 2017/085217

(56) References cited:
- MARTIN GUSTAVSSON ET AL: "Biocatalysis on the surface of Escherichia coli: melanin pigmentation of the cell exterior", SCIENTIFIC REPORTS, vol. 6, 26 October 2016 (2016-10-26), page 36117, XP055340755, DOI: 10.1038/srep36117 -& Martin Gustavsson ET AL: "Supplementary information: Biocatalysis on the surface of Escherichia coli: melanin pigmentation of the cell exterior", , 26 October 2016 (2016-10-26), XP055341490, Retrieved from the Internet: URL:http://www.nature.com/article-assets/n pg/srep/2016/161026/srep36117/extref/srep3 6117-s1.pdf [retrieved on 2017-02-02]
- Martin Gustavsson: "Influence of recombinant passenger properties and process conditions on surface expression using the AIDA-I autotransporter", , 1 January 2013 (2013-01-01), XP055341253, ISBN: 978-91-7-501770-9 Retrieved from the Internet: URL:http://kth.diva-portal.org/smash/get/d iva2:621423/FULLTEXT01.pdf [retrieved on 2017-02-01]
- JOHAN JARMANDER ET AL: "A dual tag system for facilitated detection of surface expressed proteins in Escherichia coli", MICROBIAL CELL FACTORIES, BIOMED CENTRAL, GB, vol. 11, no. 1, 3 September 2012 (2012-09-03), page 118, XP021128997, ISSN: 1475-2859, DOI: 10.1186/1475-2859-11-118
- SHUSTER VERED ET AL: "Isolation, Cloning and Characterization of a Tyrosinase with Improved Activity in Organic Solvents from Bacillus megaterium", JOURNAL OF MOLECULAR MICROBIOLOGY AND BIOTECHNOLOGY, vol. 17, no. 4, 2009, pages 188-200, XP002766709, ISSN: 1464-1801 -& DATABASE UniProt [Online] 1 July 2008 (2008-07-01), "SubName: Full=Tyrosinase {ECO:0000313|EMBL:ACC86108.1};", XP002766710, retrieved from EBI accession no. UNIPROT:B2ZB02 Database accession no. B2ZB02
- MARTIN GUSTAVSSON ET AL: "Improved cell surface display of Salmonella enterica serovar Enteritidis antigens in Escherichia coli", MICROBIAL CELL FACTORIES, BIOMED CENTRAL, GB, vol. 14, no. 1, 9 April 2015 (2015-04-09), page 47, XP021214860, ISSN: 1475-2859, DOI: 10.1186/S12934-015-0227-3
- GUSTAVSSON M ET AL: "Surface expression of -transaminase in Escherichia coli", APPLIED AND ENVIRONMENTAL MICROBIOLOGY APRIL 2014 AMERICAN SOCIETY FOR MICROBIOLOGY USA, vol. 80, no. 7, April 2014 (2014-04), pages 2293-2298, XP002766711, DOI: 10.1128/AEM.03678-13
- XU DA-YOU ET AL: "Cross-linked tyrosinase aggregates for elimination of phenolic compounds from wastewater", CHEMOSPHERE, PERGAMON PRESS, OXFORD, GB, vol. 92, no. 4, 11 February 2013 (2013-02-11), pages 391-398, XP028564261, ISSN: 0045-6535, DOI: 10.1016/J.CHEMOSPHERE.2012.12.076
- Fan ET AL: "Type V secretion systems in bacteria", , 28 February 2016 (2016-02-28), XP055340768, DOI: 10.1128/microbiolspec.VMBF-0009-2015. Retrieved from the Internet: URL:http://www.ncbi.nlm.nih.gov/pubmed/269 99388 [retrieved on 2017-01-31]

## Description

### TECHNICAL FIELD

The present invention relates to the field of water purification and to methods for performing water purification. More particularly, the invention relates to the field of purifying water from substances that are harmful to the environment, such as substances originating from pharmaceuticals, by the usage of purification means of biological origin capable of adsorbing such harmful substances.

### BACKGROUND OF THE INVENTION

Growing environmental concern continuously forces the design of sustainable processes to replace products that today are derived from fossil sources, but also to promote the development of new bioremediation methods for handling of the continued pollution of air, soil and water. Microorganisms have shown to be valuable tools in solving many of the environmental concerns in particular if the processes are economically sustainable. Continued advances in protein design and metabolic engineering today allow the directed evolution of both cells and enzymes and even include the transplantation of complete and heterogeneous pathways. Accordingly, metabolic engineering offers an important tool for introduction of new cell characteristics favoring development of sustainable processes. Still, the enclosed interior of the bacterial cell restricts external interactions with enzymes, pathways and products due to the mass-transfer barrier formed by the cell envelope, therefore offering challenges in this respect.

Pollution of the aquatic environment by pharmaceutical substances is a particular concern requiring efficient removal in wastewater treatment plants. Several of these drugs have a proven record of impairing the reproducibility of living species with fish and frogs as frequent examples²¹. The accumulation of drugs seems to be related to adsorption in particular to lipids or melanin-rich tissue present in particular in hair and the eyes^{4, 22}.

A major source for contamination is release of pharmaceutical substances through household wastewater, and efficient removal at wastewater treatment plants could therefore go a long way towards reducing the amount of pharmaceutical substances released. Several methods are undergoing investigation, including oxidation (ozonation, UV treatment, use of peroxides), membrane filtration (reverse osmosis) and adsorbents (active carbon). However, these methods are not without drawback, as they are costly and energy or resource intensive and that one cannot be sure that harmful degradation products are not formed (ozonation).

There is also a variety of conventional systems used for water purification purposes today. One example is a Membrane bioreactor (MBR) which is the combination of a membrane process, such as microfiltration or ultrafiltration, with a suspended growth bioreactor. A MBR is widely used for municipal and industrial wastewater treatment (See e.g. S. Judd, The MBR book (2006) Principles and applications of membrane bioreactors in water and wastewater treatment, Elsevier, Oxford). Furthermore, another example of a commonly used water purification system is a MBBR (moving bed biofilm reactor) which e.g. employs (polyethylene) biofilm growing on the surface of a moving carrier operating in mixed motion within an aerated wastewater treatment basin. Hence, both systems provide means for purifying waste water from pharmaceutical substances that are harmful to the environment albeit using different technology.

There is a continuous need for improved means for purifying water, particularly from pharmaceutical substances or degradation products thereof, including the need for improvements to current systems taking into account the drawbacks with presently available methods. Additional needs are also evident in view of the EU Water Framework Directive (http://ec.europa.eu/environment/water/water-framework/index en.html)

There is also a need for further cost-efficient, re-useable and environmental friendly water purification means, for the removal of pharmaceutical substances from wastewater. Particularly, there is a need for environmental friendly ways of purifying water that are based on biomaterials.

In the doctoral thesis of Martin Gustavsson (Influence of recombinant passenger properties and process conditions on surface expression using the AIDA-I autotransporter, 1 January 2013; URL:http://kth.diva-portal.org/smash/get/diva2:621423/FULLTEXT01.pdf) it is disclosed the AIDA1 expression system and its use for expression of at least nine different proteins, including three tyrosinases, one from *B.megaterium,* one from *R.etli* and one synthetic tyrosinase based on the core structure of *R.etli* tyrosinase. However, no successful expression of a tyrosinase on the surface of the bacterial cell was achieved in this experimental work.

### SUMMARY OF THE INVENTION

Herein, it was shown a way to successfully metabolically engineer a bacterial cell by introducing cell-controlled reaction pathways and by enzyme surface expression and subsequent biocatalysis allowing specific product deposition directly on the surface of said bacterial cell. Full oxidation of tyrosine by the novel synthetic enzyme (Tyr1) to a melanin or melanin-like composition on the surface of said bacterial cell created a dark pigmented exterior capable of efficiently absorbing a pharmaceutical substance, chloroquine. The broad adsorption spectrum of melanin is particularly suitable for bioremediation of pharmaceutical pollutants in wastewater being a severe environmental problem today. Hence, the partially coated bacterial cells provided herein offer a useful tool for purifying waste water, by adsorbing such substances.

Accordingly, by providing one or more bacterial cell(s) at least partially coated with a chemical-entity adsorbing composition which are capable of absorbing pharmaceutical substances on the surface thereof, the present disclosure now solves or at least mitigates some of the problems previously associated with water purification. Particularly the problems associated with purification of water from substances that are harmful to the environment, such as pharmaceutical substances are solved or at least mitigated. There is also provided an improved re-useable method for water purification in that the cells are both able to adsorb, but also release, adsorbed substances from the surface of the bacterial cells.

There is provided herein one or more bacterial cell(s) comprising a chemical-entity adsorbing composition on at least a part of the surface of said one or more bacterial cell(s). Said bacterial cells comprising a chemical-entity adsorbing composition on at least a part of the surface thereof have been produced by a method as disclosed herein. Said method comprises the introduction of an expression vector into said bacterial cells, wherein said expression vector comprises an expression cassette encoding a novel recombinantly produced tyrosinase (Tyr1) as a passenger in a Type V secretion β-autotransporter system. The tyrosinase (Tyr1) in the Type V secretion β-autotransporter system is expressed as a recombinant protein in the bacterial cell. Once the recombinant protein is expressed, the tyrosinase (Tyr1) is transported to the outside of the bacterial membrane where it is exposed externally on the cell surface towards the medium. When present on the cell surface, the tyrosinase Tyr1 was shown to be catalytically active.

The novel synthetic tyrosinase, Tyr1, presented herein, comprises the amino acid sequence of SEQ ID NO: 1. Such a tyrosinase (Tyr 1) was herein proven to possess excellent characteristics in a method for producing one or more bacterial cell(s) at least partially coated with a chemical-entity absorbing composition as further described herein.

The subsequent addition of tyrosine, or a functional equivalent thereof, to said bacterial cells triggers the formation of a chemical-entity adsorbing material on at least a part of said cell surface by the enzymatic activity of the tyrosinase present on the surface and the oxidating environment. As proven herein, the bacterial cells at least partially comprising said chemical-entity adsorbing composition on their surface are capable of adsorbing harmful substances present in water, when said water is allowed to encounter said cells.

Herein, it is shown a method which successfully results in the bacterial cells comprising a chemical-entity adsorbing composition on at least a part of a surface thereof.

In this respect, there is provided an expression vector for expressing a tyrosinase Tyr1 to be presented on the surface of a bacterial cell, said expression vector comprising an expression cassette encoding a tyrosinase (Tyr1) as a passenger in a Type V secretion β-autotransporter system, wherein said tyrosinase when expressed comprises an amino acid sequence of SEQ ID NO:1. Examples of such expression vectors are mentioned herein.

There is also provided herein a method for producing one or more bacterial cell(s) at least partially coated with a chemical entity-absorbing composition, said method comprising the steps of: a) selecting and culturing one or more bacterial cell(s) comprising an expression vector for expressing a tyrosinase (Tyr1), or a host cell comprising said expression vector as defined herein b) inducing the expression of tyrosinase (Tyr1) from said expression vector to allow presentation of said tyrosinase (Tyr1) on the surface of said one or more bacterial cell(s), c) adding tyrosine, or a functional equivalent thereof, to said one or more bacterial cell(s) to allow the formation of said chemical entity-adsorbing composition at least partially on the surface of said one or more bacterial cell(s), and thereafter d) retrieving the cells from step c).

There is also provided one or more bacterial cell(s) at least partially coated with a chemical entity-adsorbing composition obtainable by the method as disclosed herein.

There is also provided herein the use of one or more bacterial cell(s) as defined herein, for the removal of one or more chemical entities, from water, such as waste water. Said one or more bacterial cell(s) may be suspended in a solution or be present in a biofilm. The biofilm may be present on a solid support, such as a stationary or moving solid support. The chemical entity may be a pharmaceutical substance or a heavy metal compound.

There is also provided a device, such as a MBR (Membrane Bioreactor), comprising one or more bacterial cell(s) obtained herein suspended in a solution.

There is furthermore provided a device, such as a MBBR (Moving bed biofilm reactor) comprising one or more bacterial cell(s) obtained herein present in a biofilm on a solid support.

There is also provided a method for the removal of one or more chemical entities from water, such as wastewater, comprising the steps of: a) providing one or more bacterial cell(s) as disclosed herein or a biofilm comprising said cells, b) allowing water to pass through the one or more bacterial cell(s) or the biofilm comprising said cells thereby adsorbing any chemical entities present in the water thereto, c) removing any adsorbed chemical entities from the one or more bacterial cell(s) or the biofilm comprising said cells by rinsing with an aqueous composition having an acidic pH, such as a pH <7, to thereafter allow for reuse of the one or more bacterial cell(s) or the biofilm comprising said cells, and thereafter d) repeating steps b) and c).

There is furthermore provided herein a novel expression vector pAIDA1-Tyr1 (SEQ ID NO:4). Also encompassed by the present disclosure is the recombinant protein that is expressed from said vector which comprises the amino acid sequence of SEQ ID NO:3, a fragment or a variant thereof, as well as a protein comprising the amino acid sequence of SEQ ID NO:1, or a fragment thereof, and an nucleic acid sequence comprising the nucleic acid sequence of SEQ ID NO:2, SEQ ID NO:4 and SEQ ID NO:5.

### BRIEF DESCRIPTION OF THE FIGURES

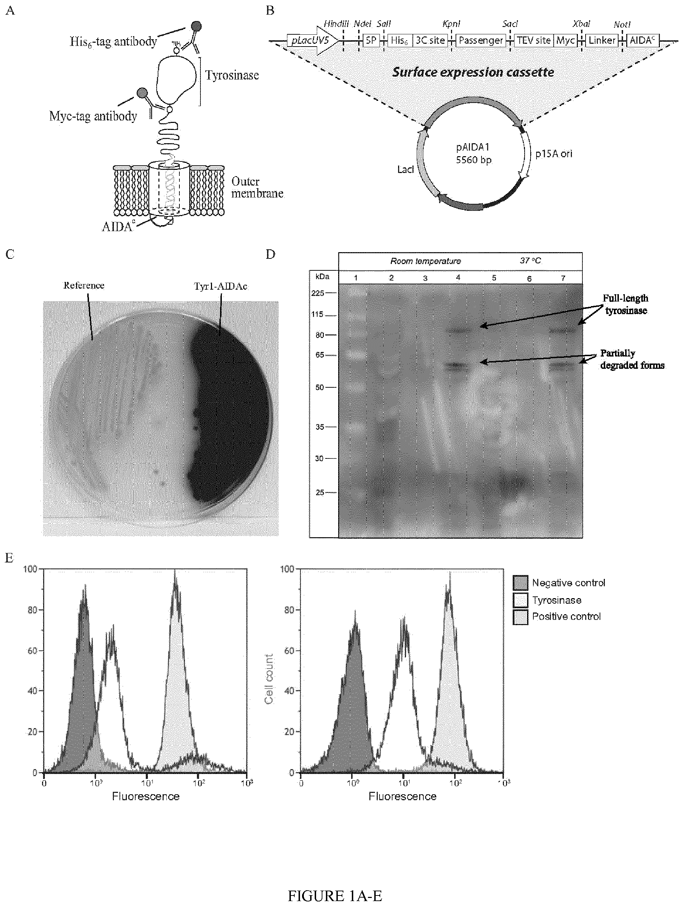

Figure 1A-E: Display of tyrosinase (Tyr1) on the surface of *E. coli*; A) Schematic representation of the imagined position on the cell surface of the enzyme and the flanking peptide tags; His6 (six histidine peptide) and Myc (peptide from the c-myc gene) both specific antibody binding. B) The surface expression cassette; C) Agar plate showing *E. coli* expression of the Tyr1-AIDA^{c} fusion protein as compared to a reference. Tyrosinase (Tyr1) is produced in an active form since melanin production is indicated by the black colour. D) Cellular protein fractions were assayed by Western blot for the presence of the Myc-tag located between Tyr1 and AIDA^{c}. The Myc-tag was absent from both the soluble and inner membrane protein fractions (lanes 2,3/5,6 respectively), while a band was visible in the outer membrane protein fraction at the expected molecular weight for Tyr1-AIDA^{c} (lanes 4 and 7). Partial proteolytic degradation was evident from bands at the approximate size of the AIDAc β-barrel. E) Probing whole cells expressing Tyr1-AIDA^{c} with fluorescent antibodies against the two epitope tags followed by flow cytometric analysis showed a clear fluorescence signal from both tags as compared to the negative control. A weaker signal was obtained from the His6-tag, likely due to the proteolysis confirmed by Western blotting of figure ID.

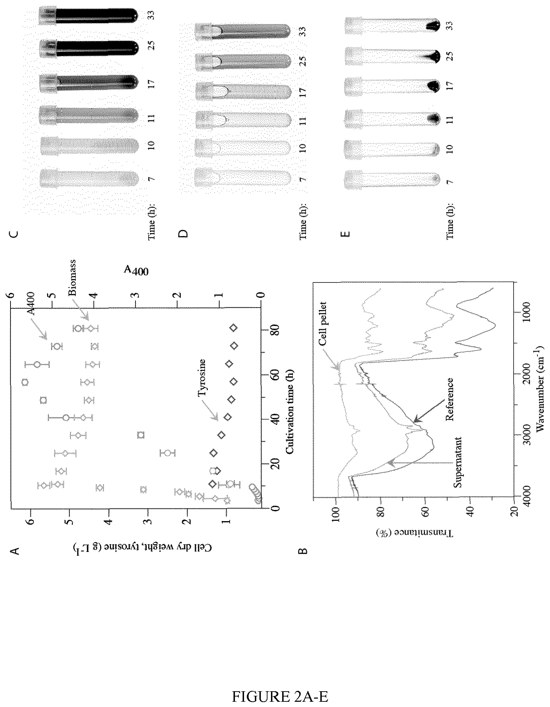

Figure 2A-E: Production of melanin by surface displayed tyrosinase Tyr 1; A) Overview of batch growth and melanin production in a lab-scale bioreactor. A steady increase in A400, corresponding to melanin pigment formation by the tyrosinase, is seen directly after the tyrosine addition at 10 hours, which takes place concomitant with sugar depletion. B) Fourier-transform infrared (FTIR) spectrograms of synthetic melanin (red) and the pigment from the cultivation medium (blue) and cell pellet (green), respectively. Clear similarities can be seen between the spectra, indicating that the formed pigment consists of melanin. C-E) Time series of pigment formation in (C) the cell suspension, (D) culture medium (D) and (E) cell pellet. The strongest colouration is visible in the cell pellet, indicating that the formed melanin remains associated with the *E. coli* cells.

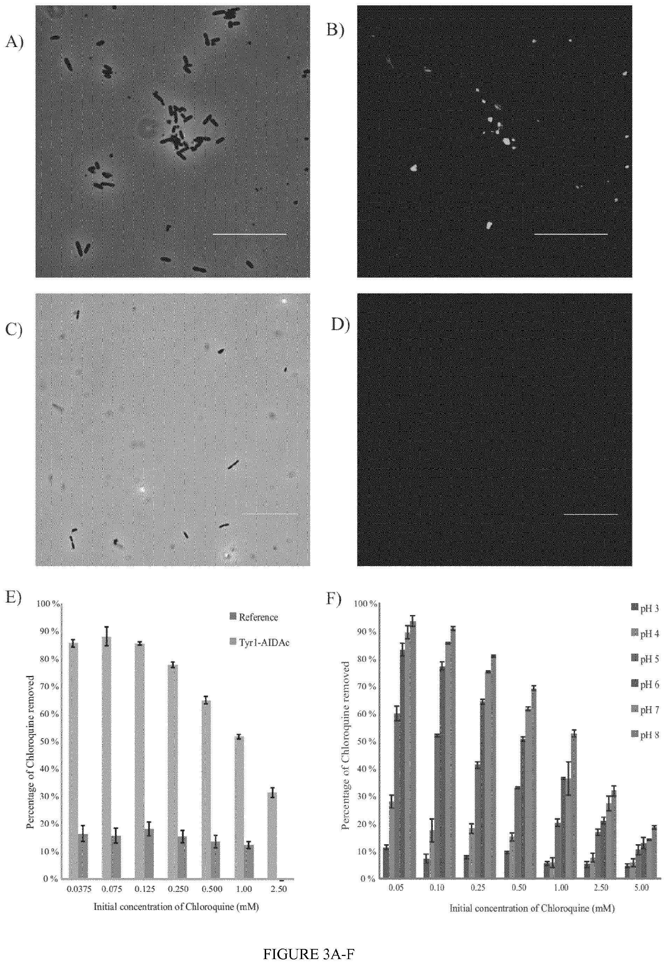

Figure 3: *E*. *coli* expressing Tyr1-AIDA^{c} deposits melanin on the surface and are able to adsorb a drug substance; *E*. *coli* were probed with fluorescent anti-melanin antibodies and analysed by fluorescence microscopy. Cells expressing Tyr1-AIDA^{c} were clearly visible (A, B), while reference cells without tyrosinase remained unstained (C, D). White bars in micrographs correspond to 20µm. Light and fluorescence microscope images to the left and right, respectively. (E) Adsorption efficiency of the antimalaria drug chloroquine to melanin coated cells. Cells expressing Tyr1-AIDA^{c} adsorbed as much as 88% of the drug at lower concentration of chloroquine (blue) while the cells expressing only the empty vector without passenger adsorbs significantly less (red). (F) Adsorbance efficiency is pH-dependent. At low drug concentrations, relevant in wastewater treatment plants, there is an approx. 90% reduction in the binding between pH 8 and 3.

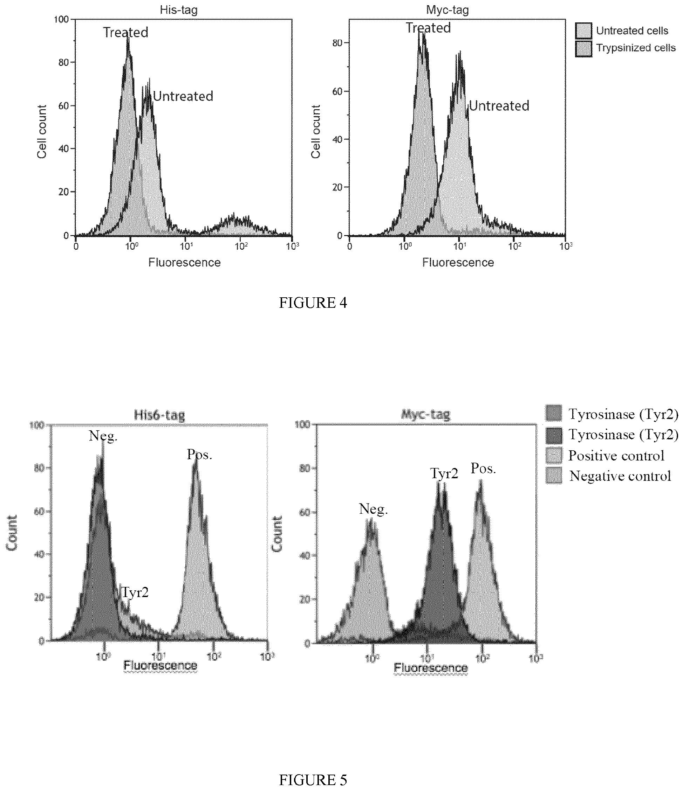

Figure 4: Flow cytometric analysis of cells after external treatment with proteases to reveal enzyme (Tyr1) present on surface. Untreated cells and trypsinized cells are shown.
Figure 5: Flow cytometric analysis of cells expressing enzymes Tyr1 or Tyr2 after exposure to proteolytic cleavage.

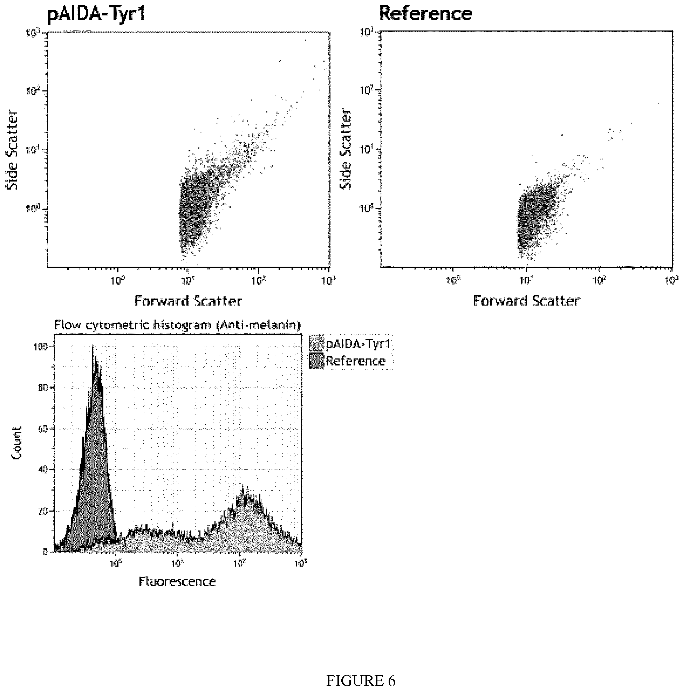

Figure 6: Flow cytometric analysis of cells comprising the vector pAIDA-Tyr1 and reference cells indicating presence or non-presence of melanin on the surface of the cells.

### DETAILED DESCRIPTION OF THE INVENTION

### Definitions

The terms used herein are intended to have the same meaning usually provided in the art, but are also further explained in the below.

"Expression" is used to mean the production of a protein from a gene and refer herein to the genes of and comprises the steps of "the central dogma" i.e. the successive action of transcription, translation and protein folding to reach the active state of the protein all as described in standard biochemistry textbooks. All these steps and elements are controlled in the bacterial cell by multiple means. Although the general steps used for expression of genes are well known to a person skilled in the state-of-the-art, the combination of variants of these steps is generally designed for each new DNA sequence to be expressed from a plasmid.

An "expression vector" as defined herein, which may also be referred to as an expression construct, comprises gene sequences to achieve protein expression in cells. The expression vector herein is used to introduce a specific gene into a cell, to thereafter direct the cell machinery for protein synthesis to produce the protein encoded by the gene. A bacterial plasmid is an example of an expression vector encompassed by the present disclosure.

A "plasmid" is a small circular extra-chromosomal DNA molecule that can replicate independently of the cell and are found in bacteria. Plasmids are often used as vectors for molecular cloning i.e. to transfer and introduce selected DNA to a host cell. Plasmids are built-up from specific and necessary elements and may contain genes that can be homo- or heterologous to the bacterial host cell. Plasmids contain e.g. always an origin of replication and most often a gene for specific antibiotics resistance. The typical elements of a plasmid used for gene transfer in *Escherichia coli* can be found in the review by Makrides SC (Strategies for achieving high-level expression of genes in Escherichia coli, Microbial Rev 60:512-538, 1996). The words "vector" or "plasmid vector" and "expression vector" are often used as synonyms for "plasmid".

A "β-autotransporter system" as referred to herein, comprises the family of β-autotransporters (Leyton et al, 2012), including the β-autotransporter AIDA (Adhesin Involved in Diffuse Adherence) herein called AIDA-I (Benz and Schmidt, 1992), which provides a specific mechanism to present a protein on the surface of a bacterial cell. The β-autotransporter system is a part of the Type V secretion system, and is therefore also referred to herein as the Type V secretion β-autotransporter system. It may also be referred to as the β-autotransporter system only. Said protein is called a "passenger" in said Type V secretion β-autotransporter system and the passenger herein is tyrosinase (Tyr1).

With an "expression cassette" is herein meant the specific part of the sequence of an expression vector that comprises the genes for expression of a protein, said expression cassette herein comprising a β-autotransporter system including the passenger tyrosinase Tyr 1 and appropriate regulatory sequences. An example of such an expression cassette is shown in figure IB. The novel tyrosinase protein presented herein, was successfully expressed and proven active on the cell surface when a β-autotransporter system was used, herein exemplified by AIDA-I and the plasmid pAIDAI-Tyr1.

By a "recombinant" protein as mentioned herein, is meant a synthetic protein manufactured by DNA manipulation, which are techniques well-known to the person skilled in the art.

By an amino acid sequence having a particular "identity" to a nucleic or amino acid sequence it is intended herein that such a sequence may include up to 5% "modifications" of the original amino acid reference sequence, or up to 20% "modifications", of the original nucleic acid reference sequence. Such a modification may be in the form of addition of one or more nucleic acid(s) or amino acid(s) to the reference sequence. These one or more amino acids or nucleic acids may have been intentionally added to one or both ends of the reference sequence or they may have been added within the sequence, all resulting in a longer nucleic acid or amino acid sequence than the reference sequence. Furthermore, a modification of a particular sequence may also arise where nucleic acids or amino acids have been removed/deleted/exchanged within the sequence but wherein the full sequence of the modified sequence still comprises at least 95% identity with the original sequence. Hence, to correspond to at least 95% identity to SEQ ID NO: 1, up to 15 amino acids may have been modified in SEQ ID NO: 1 (by addition, and/or deletion/removal/exchange), such as 1, 2, 3, 4, 5, 6, 7, 8, 10, 11, 12, 13, 14, or 15 amino acids. The same applies to SEQ ID NO: 1 when being part of SEQ ID NO:3 (in an β-autotransporter system). There is also provided a tyrosinase having at least 96, 97, 98 or 99% identity with SEQ ID NO: 1, or where 1 or 2 amino acids have been modified in said sequence. Still, such a modified version with a particular identity to Tyr1 (SEQ ID NO:1) still comprises amino acids 1-4 of SEQ ID NO: 1 in positions 1-4 as further explained herein.

To correspond to at least 80% identity to SEQ ID NO:2, up to about 180 nucleic acids may have been modified in SEQ ID NO:2. There is also provided a tyrosinase which is encoded by a sequence having at least 85, 90, 95, 96, 97, 98 or 99% identity with SEQ ID NO:2.

Encompassed by the present disclosure are of course also conventional codon-optimized versions of the nucleic acid sequences presented herein, wherein the identity of the final protein is the same but nucleic acid sequences have been optimized e.g. for a certain host cell, such as *E.coli.*

Accordingly, the tyrosinase (Tyr1) as disclosed herein may differ from SEQ ID NO:2 by one or more modifications, such as i.e. by deletion or addition of one or more nucleic acids, such as by a point mutation. Still, such a modified version of Tyr1 comprises the amino acids of SEQ ID NO: 1.

The percentage of identity between two sequences may e.g. be determined by the sequence alignment program BLAST (Altschul SF, Gish W, Miller W, Myers EW, Lipman DJ) (http://blast.ncbi.nlm.nih.gov/Blast.cgi).

A "fragment" of a protein as defined herein comprises a protein having an amino acid sequence which has been shortened with reference to the original protein, such as by a total of 1-15 amino acids in one or both ends of the protein, and which still results in a protein having substantially the same biological function as the protein comprising the amino acid sequence of SEQ ID NO:1. Still, such a fragment of Tyr1 still comprises amino acids 1-4 of SEQ ID NO: 1 in positions 1-4.

When a modified protein is expressed, such as having a particular sequence identity as described herein and/or being a fragment of the original protein, this will result in a protein having substantially the same biological function as the protein comprising the amino acid sequence of SEQ ID NO: 1 or encoded by the nucleic acid sequence of SEQ ID NO:2, SEQ ID NO:15 or SEQ ID NO:5 i.e. being a functional equivalent thereof. Herein, it is intended a tyrosinase which when expressed in a Type V secretion β-autotransporter system is capable of being transported to the surface of a bacterial cell where it thereafter can execute its function as a tyrosinase in the context of this disclosure i.e. catalyzing a reaction with tyrosine as the substrate, or a functional equivalent thereof, when tyrosine is added to the bacterial cell(s) exposing the active tyrosinase. Herein, Tyr1 was shown to be particularly suitable for this purpose. It has been shown that the first amino acids in a protein are important for translation in a bacterial cell, and particularly in an *E.coli* cell (de Valdivia, E. I. G.; Studies on translation initiation and gene expression in Escherichia coli ISBN 91-7155-214-6 PhD Thesis Stockholm University; Ernesto et al. A codon window in mRNA downstream of the initiation codon where NGG codons give strongly reduced gene expression in Escherichia coli, Nucleic acids research, 2004, Vol.32, No.17; Ernesto et al., Abortive translation caused by peptidyl-tRNA drop-off at NGG codons in the early codon region of mRNA, FEBS Journal 272 (2005) 5306-5316).

A "promotor" is a region of DNA which initiates transcription of a gene.

A "host cell" as referred to herein, relates to a bacterial cell which has been transformed by an expression vector as disclosed herein. A host cell herein is a bacterial cell. In the example given herein, the host cell is *Escherichia coli,* or *E. coli.*

When it is referred to that "at least a part of the surface" of one or more bacterial cell(s) comprises chemical-entity adsorbing composition, this is intended to mean that a sufficient part of the surface of the cell comprises the chemical entity adsorbing composition to allow the adsorption of chemical entities, as mentioned herein, thereto. An example of a successful adsorption is provided in the experimental section.

A "biofilm" comprises an aggregate of microorganisms in which cells adhere to each other and/or to a surface. Cells in a biofilm may be embedded within a self-produced matrix of extracellular polymeric substance (EPS).

### Detailed description

Accordingly, there is provided herein a novel and inventive approach to water purification including the use of a novel tyrosinase (Tyr1) for cell-surface expression and subsequent catalysis of tyrosine and formation of a chemical-entity adsorbing composition on said surface. The method provided herein comprising the combination of a successful expression of a novel active tyrosinase (Tyr1) on the surface of a bacterial cell, and the subsequent formation of a chemical-entity adsorbing composition on at least a part of a surface of said bacterial cell was proven to result in an efficient material (a coated bacterial cell) for water-purification purposes.

The specific research challenge of the present disclosure was threefold: (1) the design of the expression system and the transport of tyrosinase (Tyr1) to the outer part of the outer membrane with a proven exposure to the environment and in an active configuration, (2) from this enzyme, provide the conditions necessary for catalysis of the polymerisation reaction and complete oxidation to the polymer and (3) the use of the inherent characteristics of melanin, such as its well-known adsorption of e.g. pharmaceuticals⁴ to prove the benefits of the surface deposited product melanin. This challenge was successfully for the first time completed by the present disclosure by using a tyrosinase, herein named Tyr1 in a Type V secretion β-autotransporter system. Tyr1, a synthetic protein, was shown to be a particularly suitable tyrosinase for the purpose of the present disclosure, as illustrated by the experimental results presented herein.

Initially, two enzymes (Tyr 1, Tyr 2) were introduced as passengers into the β-autotransporter system autotransporter AIDA (Adhesin Involved in Diffuse Adherence). The vectors were subsequently introduced into an OmpT-negative strain to avoid the AIDA-inherent cleavage of the passenger from the surface⁸. Expression of Tyr1 and subsequent melanin formation was initially detected already on agar plates as blackish colonies (figure 1C). To understand the efficiency of melanin formation the degree of localization of the enzyme on the surface was investigated. Western blotting of soluble (S), inner membrane (IM) and outer membrane (OM) cell fractions revealed the expected 83 kDa band only in the OM fraction, thus confirming the presence of a full-length Tyr1-AIDAc fusion protein (likely to about correspond to the protein of SEQ ID NO:3) at this location (figure ID). Apart from the full-length protein, smaller proteolytic fragments were also detected and in particular, a strong band at 60 kDa, correlating to the size of AIDA^{c} without the passenger protein. This is in accordance with previous findings^{7, 13, 14} and shows that inherent autoproteolytic cleavage is not yet fully removed. To determine the correct tyrosinase orientation in the OM flow cytometric analysis was performed. A strong signal from the Myc tag antibody (figure IE), positioned closest to the surface with the enzyme further outwards, was given and proved the right orientation i.e. directed away from the outer membrane. The weaker His6 tag signal, further exterior, suggests that the full-length protein is present but a fraction is proteolytically degraded, which is in agreement with the Western blot data (figure 1C). To further confirm the surface localisation, cells were externally treated with proteases to supposedly remove the enzyme if present on the surface, which indeed resulted in a clear decrease in both fluorescence signals (figure 4).

In contrast to Tyr1, Tyr2 was found to be inefficiently expressed on the cell surface as verified both by flow cytometry and Western blot analysis and in particular, the His6-tag is proteolytically cleaved (figure 5). Without wishing to be bound by theory, this is partly likely to be due to its larger size and the presence of cysteine residues^{7, 9}. To try to remove these obstacles, homology modelling based on smaller tyrosinases was used and revealed extensions¹⁶ both in the C- and N-terminal and these were engineered to give a minimised *R. etli* domain. The modified Tyr2 (p-AIDA1-Tyr2 core) was more efficiently expressed, but did still not to the level of Tyr1. Hence, Tyr1 was identified as a particularly suitable enzyme for the present purpose and was selected for further analysis.

Once tyrosinase (Tyr1) expression was verified the biocatalysis reaction was introduced by addition of tyrosine. Since melanin polymerisation from tyrosine is subjected to several constraints process conditions were screened. This was done in bioreactors since constant pH, temperature and oxidative conditions were imperative qualities needed for the polymerisation. Furthermore, tyrosine is not readily soluble at pH7 in the quantities needed for the processing and had eventually to be solubilised at pH 10.5 in a stock solution. After the final process was established cells were grown in batch until the carbon source was depleted where after tyrosine was added at dissolved oxygen concentrations above 30%. The biomass growth and melanin formation were followed by frequent sampling throughout the cultivation 17 (figure 2A). At the end of the cultivation a strong dark colour had developed in the reactor and cell harvest revealed that the formed pigment was present both in the cell pellet and supernatant (figure 2C-E). Repeated washing of the cell pellet led however only to marginal melanin extraction and no visible colour shift was seen, indicating that a large part of the formed melanin remained associated with the cells (data not shown).

To verify that the pigment was indeed melanin, the dark substance was extracted from the cell pellet and this and the supernatant sample were both compared to a reference sample of synthetic melanin (figure 2B). These all showed a similar spectrum and the overall patterns were well in agreement with previous melanin data^{18, 19}.

As the formed melanin appeared to remain associated to the cells it was proceeded to understand and prove its surface location. As it seemed quite unlikely that the large polymer could pass into the cell interior it was hypothesized that it might form a layer coating the cell. To test this hypothesis pigmented cells were incubated with anti-melanin antibodies and analysed with florescent microscopy. This revealed that melanin was clearly located on the surface (figure 3 A and B), while the negative control, cells lacking the pigment, remained unstained (figure 3 C and D). The difference compared to the reference is also clearly verified by flow cytometry (figure 6, bottom). It might be argued that there is a possibility to introduce an error by measurement of free melanin particles i.e. those not attached to the cell surface but this is opposed by the fact that both reference and melanin expressing cells have the same side- and forward scattering profile which means that they are of equal size and characteristics (figure 6).

The adsorption capacity with respect to a selected drug substance was thereafter investigated. Chloroquine, an antimalarial drug that has a well-established affinity for melanin²² was chosen for this purpose. It is shown herein that cells coated with melanin (catalysis by Tyr1) successfully adsorbed up to 88% of the chloroquine in particular at concentrations relevant of most pharmaceutical pollution in wastewater i.e. nanograms to a few milligrams per liter²⁰, while only minor binding occurred in reference samples (figure 3F).

Since this suggested a future in bioremediation processes the possibility also of regeneration of the system was explored i.e. the conditions for desorption of the pharmaceutical and it was found that the binding affinity was largely destabilised by decreased pH (figure 4). The regeneration could thus be accomplished by a simple washing step that would also very simply retrieve the adsorbed pharmaceutical substances.

In conclusion, it has successfully been shown herein the change the characteristics of a wild type E. *coli* (K12) strain by introduction of a reaction pathway on the cell exterior, here resulting in surface deposition of melanin, or a melanin-like substance. It is further shown that this black cell could be used for removal of a drug substance under conditions that very much resemble the situation in the outgoing water of a wastewater treatment plant i.e. a situation where the water is depleted of nutrients, particles and general pollution as it is aimed for release to a recipient. In this case, the cells act as passive adsorbents and require no specific conditions for being in full operation. Furthermore, it is shown a simplified regeneration procedure, which might add to the finding of an economic process solution.

The present disclosure provides a way to solve of one of the most urgent ecological problems today, the accumulation of drug substances in aquatic environments due to household consumption.

Accordingly, the present inventors have now surprisingly made it possible to produce one or more bacterial cell(s) at least partially coated with a chemical-entity adsorbing composition, wherein said bacterial cells have been shown to efficiently adsorb harmful chemical substances, i.e. substances that are harmful to the environment when present in the water, such as pharmaceutical substances. These cells hence finds a use in purifying water, such as waste water, from these substances.

This was made possible by identifying a novel tyrosinase (Tyr1) which when expressed in an Type V β-autotransporter system, e.g AIDA, could be presented as a biologically active tyrosinase on the surface of a bacterial cell, and which thereafter, as described herein, further made it possible to generate bacterial cells at least partially coated with a chemical-entity forming composition.

The expression of Tyr1 in a Type V secretion β-autotransporter system was surprisingly shown to result in the transport of Tyr1 over the inner membrane, through the periplasm including the cell wall and through the outer membrane, all without premature folding of the protein and resulting in a large portion of full length protein on the cell surface. Thereafter, Tyr1 was exposed in such a way that it was facing the exterior and was further not embedded in the outer membrane. In addition, it was shown to be folded in such a way that it was active i.e. that it can use utilise its substrate tyrosine, or a functional equivalent thereof, in such a way that it performs its enzymatic reaction at high catalytic activity.

In addition, the method for producing the bacterial cell(s) presented herein was designed to be tuned in such a way that the production of the tyrosine enzyme is not too fast which will lead to premature folding and/or degradation. The tuning involved using a suitable amount of IPTG, choosing a suitable promotor (medium strong) and using a low plasmid copy number (about 10-20 copies). This in combination with Tyr1 as a passenger protein in a Type V secretion β-autotransporter system made it possible to produce bacterial cells at least partially coated with a chemical-entity adsorbing material.

Hence, herein, it is presented at least partially chemical-entity adsorbing coated or melanin-coated bacterial cells capable of adsorption of pharmaceutical substances. There is provided herein the display of an active novel tyrosinase, Tyr1, on the surface of a bacterial cell, exemplified by *Escherichia coli,* which resulted in at least partially chemical-entity coated or melanin-coated bacterial cells (*E. coli*) cell upon the addition of tyrosine thereto. These cells are further shown to efficiently adsorb a model pharmaceutical substance, chloroquine.

Accordingly, to achieve the purposes of the present disclosure there is provided herein an expression vector for expressing a tyrosinase (Tyr1) to be presented on the surface of a bacterial cell, said expression vector comprising an expression cassette encoding a tyrosinase (Tyr1) as a passenger in a Type V secretion β-autotransporter system, wherein said tyrosinase when expressed comprises a protein comprising an amino acid sequence of SEQ ID NO:1.

Accordingly, whenever an "expression vector" is referred to herein what is intended is a vector from which it is possible to express, by firstly transcribing, the gene encoding the protein and thereafter translate the RNA from said transcription into a protein. An example of an expression vector herein is a plasmid. The expression vector herein is usually a DNA vector. An example of a Type V β-autotransporter system used herein is AIDA-I, which is illustrated in figure 1B together with a vector backbone.

When the expression cassette encoding a tyrosinase, Tyr1, as a passenger in a Type V secretion β-autotransporter system is referred to herein this also means that the expression cassette in addition to the recombinant tyrosinase encodes additional proteins, such as signal peptides, linker peptides or other protein tags or the like that aids in the transport, positioning and/or detection of the tyrosinase in the cell membrane. These entities form part of the β-autotransporter system as shown in figure IB. The expression cassette further comprises regulatory sequences as previously mentioned herein. Hence, there is provided herein an expression vector wherein said expression cassette encodes or comprises one or more of the following components: the promotor pLacUV5, a signal peptide, a linker region, a His-tag site, a Myc-Tag site, a 3C site, and/or a TEV site, or as illustrated in figure IB.

A Type V secretion β-autotransporter system in the present context, refers to an abundant group of translocators that use a specific set of ingoing elements in a vector cassette which renders it capable of transporting a recombinant passenger protein (herein the tyrosinase Tyr1) through a specific translocator domain (herein exemplified by AIDA^{c}) inserted into the cell outer membrane and thereafter exposing/presenting the passenger protein on the surface of said cells or releasing it to the medium. Herein, an example of such a Type V β-autotransporter is displayed by the AIDA-I system.

Thus, the present disclosure extends to the usage of other Type V β-autotransporter proteins (systems) other than AIDA-I, such as exemplified in www.pfam.sanger.ac.uk, accession PF03787 (Pallen MJ, Chaudhuri RR, Henderson IR. Genomic analysis of secretion systems. Curr Opin Microbiol. 2003;6: 519-527.) As of today, 8209 sequences are listed. A specific feature is the generality of some of the Type V β-autotransporter parts i.e. the translocator domain (AIDA^{C} in the AIDA-I system) which in many cases is interchangeable with other β-autotransporters due to the same functionality. This means that autotransporter vector parts may be possible to move from one system to another and used to promote translocation.

A novel recombinant tyrosinase, Tyr1, as presented herein, was proven to possess the accurate characteristics to successfully allocate to the surface of the bacterial cell by using the Type V β-autotransporter system. It was also proven to be active, i.e. possessing tyrosinase activity, once exposed on the exterior of the cell membrane.

Such a tyrosinase, Tyr1, was particularly useful in the context of the present disclosure making it possible to provide bacterial cells at least partly covered with a chemical-entity adsorbing composition once tyrosine, or a functional equivalent, is added to the cell culture. Such bacterial cells were thereafter proven to efficiently absorb a pharmaceutical substance from water.

Furthermore, it is also disclosed herein a tyrosinase (Tyr1) wherein the nucleic acid sequence as illustrated in SEQ ID NO:2 has been modified in a manner as previously described herein.

Furthermore, there is also provided an expression vector, wherein said tyrosinase (Tyr1) consists of an amino acid sequence of SEQ ID NO: 1. There is also provided a tyrosinase (Tyr1) wherein said tyrosinase may be encoded by a nucleic acid sequence comprising the sequence of SEQ ID NO:2, or a sequence having at least 80% identity, such as at least 85%, 90%, 91%, 92%, 93%, 94%, 97 % or 98% identity with SEQ ID NO:2 still encoding a functional equivalent of the protein. Accordingly, this also includes e.g. codon-optimized versions of the nucleic acid sequences. There is also disclosed herein an expression vector wherein said tyrosinase (Tyr1) is encoded by a nucleic acid sequence consisting of the nucleic acid sequence of SEQ ID NO:2 or SEQ ID NO: 15.

There is also provided herein an expression vector wherein said β-autotransporter system is AIDA-I (Adhesin Involved in Diffuse Adherence).

There is also provided an expression vector wherein said surface expression cassette encodes the protein tyrosinase Tyr 1 as previously defined in the β-autotransporter system AIDA-I (SEQ ID NO:3).

There is further provided an expression vector wherein the recombinant tyrosinase Tyr 1 in the Type V secretion β-autotransporter system AIDA-I is encoded by a nucleic acid sequence comprising SEQ ID NO:5, or a sequence having at least 80% identity with SEQ ID NO:5, such as at least at least 85%, 90%, 91%, 92%, 93%, 94%, 97 % or 98% identity with SEQ ID NO:5.

There is also provided herein an expression vector which is pAIDA1-Tyr (SEQ ID NO:4), or expression vector having at least 80% sequence identity with the sequence of SEQ ID NO:4, such as a vector having at least 85%, 90%, 91%, 92%, 93%, 94%, 97 % or 98% identity with SEQ ID NO:4. There is also provided herein an expression vector which is pAIDA1-Tyr (SEQ ID NO: 4) and which comprises or consists of the nucleic acid sequence of SEQ ID NO:4.

It is also envisaged a variant of pAIDA1-Tyr (SEQ ID NO:4) where the vector backbone (excluding the expression cassette comprising the Type V secretion β-autotransporter system) has been modified in the manner that some structural parts thereof have been exchanged for equally suitable structural parts in a functional context. For example, an origin of replication or the antibiotics resistance may be changed to another one but still provide the same function. Furthermore, it is also encompassed the modification of any non-coding sequences, such as one or more of the regulatory part(s) of the vectors, such as the translation initiation site of the vector etc. These are modifications to the vector backbone which the skilled person can do without undue experimentation. Such expression vectors are also within the scope of this disclosure.

The expression vector provided herein may be a plasmid, such as a low-copy and/or a plasmid comprising an inducible promoter or a promotor for constitutive expression. A low copy plasmid is a plasmid generating about 10-50 copies, such as 10-20 copies, of the plasmid in the cell and is being particularly suitable in the present context to generate a sufficient amount of the tyrosinase Tyr1.

An plasmid comprising an inducible promoter or promotor for constitutive expression is also particularly suitable in the present context, as the transcription and subsequent expression (translation) of the protein may thereby be controlled allowing for controlled expression of the protein when the expression vector has been introduced into the bacterial cells. It may be so that it is preferable to await a particular stage of cultivation of the bacterial cells before the expression (transcription) of the surface expression cassette is induced. This may be made possible due to the usage of such a controlled system. Furthermore, the synthesis of the protein will also be slower when the expression can be controlled in this manner, which is particularly preferred due to that otherwise one may compromise with the structure and function, particularly proteolysis and folding of the tyrosinase.

Induction of expression (transcription) can be performed by the addition of IPTG (Isopropyl β-D-1-thiogalactopyranoside), or by lactose to the bacterial culture. IPTG or lactose then bind the repressor of the lactose operon and thereby initiates the transcription of the genes in a known manner.

The bacterial cell culture medium used in the present context may be a defined minimal salts medium. Glucose is then added as the carbon source to reach a certain amount of cell mass where after the induction takes place.

Particularly, the expression vector disclosed herein comprises a promotor which is not dependent upon the sugar concentration in the cell medium used for cultivating the bacterial cells. An example of such a promotor is the promotor pLacUV5, but other promoters are also envisaged.

There is further provided herein a host cell comprising an expression vector as disclosed herein. There is provided such a host cell which is a bacterial cell. Furthermore, such a host cell, such as an E.coli cell, suitably lacks OmpT (an aspartyl protease found on the outer membrane of *Escherichia coli*) to avoid the presence of a protease that may cleave AIDA in the outer membrane of the cell. The host cell may be an *E.coli* cell, such as an E.coli K12 strain, such as E.coli K12 0:17. The host cell may also be the strain E.coli 0: 17K12Δ*ompT*²³*.* In the later, the gene for OmpT has been mutated by general means generating a bacterial cell that lacks this protease.

There is also provided herein a method for producing one or more bacterial cell(s) at least partially coated with a chemical entity-adsorbing composition, said method comprising the steps of: a) selecting and culturing one or more bacterial cell(s) comprising an expression vector as disclosed herein, or an host cell comprising an expression vector as disclosed herein, b) inducing the expression of tyrosinase (Tyr 1) from said expression vector to allow presentation of said tyrosinase (Tyr1) on the surface of said one or more bacterial cell(s), c) adding a suitable amount of tyrosine, or a functional equivalent thereof, to said one or more bacterial cell(s) to allow the formation of said chemical entity-adsorbing composition at least partially on the surface of said one or more bacterial cell(s), and thereafter d) retrieving the cells from step c).

A functional equivalent of tyrosine is referred to herein as a composition which will have substantially the same effect as tyrosine when added to the tyrosinase on the surface of the one or more bacterial cell(s). This means that it will be catalyzed by the tyrosinase Tyr1 in the same manner as tyrosine generating one or more bacterial cell(s) which are at least partially covered with a chemical-entity adsorbing composition, such as melanin or a structurally related variant thereof.

A chemical-entity adsorbing composition herein is referred to due to that even though it is known that tyrosinase catalyzes tyrosine to melanin, it may be so that other structural components are formed during the catalysis of the tyrosine at the cell surface generating an adsorbing composition which does not entirely consist of melanin, even though a part of the composition may comprise melanin. Examples of such additional structural components are intermediates of melanin. This is however said without wishing to be bound by theory.

In such a method as presented herein, the culturing of the cells may be performed at a pH of about 7 to 8, such as at about pH 7.5. Maintaining the pH in the cell culture within such ranges was shown to improve the activity of the tyrosinase when positioned in the cell membrane and exposed on the exterior of the cell surface. Furthermore, adjusting the pH in the cell culture was shown to improve the formation of the chemical-entity absorbing composition on the surface of the bacterial cells.

It was also shown that the administration mode in the form of timing of and/or amount of induction agent, such as IPTG, added to the cell culture had an effect on the formation of the chemical entity-absorbing composition on at least a part of the surface on at least one or more bacterial cell(s). Accordingly, the activation in step b) of the method may be performed by adding IPTG or lactose to the cell culture, such as in an amount of about 200 µM per activation, and further by adding copper (Cu2+), such as in the form of CuSO₄. CuSO₄ may be in a concentration of about 10µM, as exemplified herein. Hence, in step b) the tyrosinase once expressed is also activated by the addition of Copper (in the form of CuSO₄).

There is also provided a method wherein the expression of the tyrosinase Tyr1 is constitutive, meaning that it is under control by a constitutive promoter.

There is also provided a method wherein expression of the tyrosinase Tyr1 is allowed to continue during a minimum of two cell generations after the induction in step b), such as 2, 3, 4, 5 or 6 generations. This is particularly useful when attempting to generate increased amounts of chemical-entity adsorbing material on the cell surface.

There is furthermore provided a method as disclosed herein wherein tyrosine (Tyr1) is in the form of a powder composition. In addition to the other features of the present method, it was also shown that adding the tyrosine in a powder format could improve the formation of the chemical entity-adsorbing composition on at least a part of the surface on at least one or more bacterial cell(s).

The tyrosine, or a functional equivalent thereof, may be contained in an aqueous basic composition or solution having a pH of about 9-12, such as about pH 10, or about pH 10.5 The basic pH of this composition was shown to increase the solubility of tyrosine which in turn resulted in increased access to tyrosine providing a higher binding to melanin. The method may be performed in an oxidative environment, wherein the DOT (Dissolved Oxygen Tension) is at least about > 30%.

As previously mentioned herein, the chemical entity-adsorbing composition may comprise melanin, an intermediate thereof, a functional equivalent or a structurally related variant thereof. The chemical entity-adsorbing composition may hence comprise melanin in combination with other compounds aiding in the adsorption of chemical entities to the bacterial cells.

The chemical entities in a method as disclosed herein comprise one or more pharmaceutical substances or compound(s), such as small molecules, or one or more degradation products thereof. Hence, a chemical-entity adsorbing composition may also be a pharmaceutical substance-adsorbing composition.

An example of a pharmaceutical substance that binds to a bacterial cell at least partially coated with a chemical-entity adsorbing composition is chloroquine, but the present disclosure is not limited thereto. The heterogeneity of melanin affinity is rather large. Other examples of chemical entities in the present context are organic amines and metal ions. Further examples comprise toxins (beta-N-methylamino-L-alanine, BMAA, alfatoxin B1), various drugs (e.g. phenothiazines, tricyclic antidepressants, aminoglycosides, and chlorpromazine), other compounds (herbicides, illicit drugs (amphetamine), alkaloids and metals.⁴

An additional example of a chemical-entity binding to melanin or a melanin-like composition is a heavy metal compound, such as divalent cations (Larsson et al, Pigment Cell Research, 1993; Interactions between chemicals and melanin). Other metals include for instance: Pb, Ni, Fe.⁴

There is also provided herein one or more bacterial cell(s) at least partially coated with a chemical entity-adsorbing composition obtainable by the method as disclosed herein. There is also provided the use of said one or more bacterial cell(s) at least partially coated with a chemical entity-adsorbing composition as disclosed herein for purifying waste water, e.g. by removing one or more chemical entities from said waste water as exemplified herein.

Such one or more bacterial cell(s) comprising said bacterial cells may be present in a biofilm present on a solid support, or they may be suspended in a solution (suspended growth). The solid support may be in the form of a stationary solid support or a moving solid support.

Examples of conventional systems for purifying water are MBR (Membrane Bioreactor) and MBBR (Moving Bed Biofilm Reactor), which may encompass said one or more bacterial cell(s). Hence, there is also provided herein a device for water purification, such a reactor, comprising said one or more at least partially coated bacterial cell(s).

In an MBR, cells are grown as suspended in a bioreactor and contained in the system with a membrane. New polluted water is added continuously and substances are captured by the cells and thereafter allowing the clean water to pass through the membrane. An example is a Hollow fibre reactor.

In an MBBR (Moving Bed biofilm reactor) cells are growing as a biofilm on a moving solid support (e.g. on particles). The moving media is maintained in the reactor by the same principle as for MBR. An example of a moving solid support is a floating plastic substrate.

An example of a stationary solid support (or bed) is a trickling filter or a biotower. Stationary particles e.g. in the form of rock or plastic containing biofilm growth bacteria are packed in a bed or a column, respectively. Water is introduced in the top, trickles through the bed and clean water thereafter leaves in the bottom of the bed.

There is furthermore related to herein the use of one or more bacterial cell(s) obtained herein, wherein said cells are suspended in solution or when said cells are present in a biofilm. The biofilm may be present on a solid support, such as a stationary solid support or a moving solid support. As previously mentioned herein, the chemical entity adsorbed by a bacterial cell at least partially coated with a chemical-entity adsorbing composition may be pharmaceutical substance, or a heavy metal compound. In addition, there is provided a device, such as a MBR (Membrane Bioreactor), comprising one or more bacterial cell(s) as disclosed herein suspended in a solution.

Furthermore, there is provided a device, such as a MBBR (Moving bed biofilm reactor) comprising one or more bacterial cell(s) as disclosed herein present in a biofilm on a solid support.

There is further provided herein a method for the removal of one or more chemical entities from water, such as wastewater, comprising the steps of: a) Providing one or more bacterial cell(s) or a biofilm comprising said cells as disclosed herein, b) allowing water to pass through the one or more bacterial cell(s) or the biofilm comprising said cells as disclosed herein thereby adsorbing any chemical entities present in the water thereto, c) removing any adsorbed chemical entities from the one or more bacterial cell(s) or the biofilm comprising said cells as disclosed herein by rinsing with an aqueous composition at an acidic pH, to thereafter allow for reuse of the one or more bacterial cell(s) or biofilm comprising said cells, and thereafter d) repeating steps b) and c).

In such a method, the one or more chemical entities may be pharmaceutical substances, or heavy metal compounds, or degradation products thereof. In such a method the one or more chemical entities comprise substances that have affinity to the chemical entity-adsorbing composition e.g. substances that are harmful to the environment.

There is furthermore provided herein a recombinant protein comprising the amino acid sequence of SEQ ID NO: 1. There is furthermore provided an nucleic acid sequence comprising the nucleic acid sequence of SEQ ID NO:2, a fragment thereof, or a functional equivalent thereof encoded by a nucleic acid sequence having 80% identity therewith, such as at least 85%, 90%, 91%, 92%, 93%, 94%, 97 % or 98% identity therewith.

There is furthermore provided herein a protein comprising the amino acid sequence of SEQ ID NO:3, a fragment thereof, or a functional equivalent thereof, having at least 95% identity with SEQ ID NO:3, such as 96%, 97%, 98% or 99% identity, as explained herein.

There is furthermore provided herein an expression vector comprising the nucleic acid sequence of SEQ ID NO:4 or comprising a nucleic acid sequence having 80% identity therewith, such as at least 85%, 90%, 91%, 92%, 93%, 94%, 97 % or 98% identity therewith and/or a vector wherein parts therein have been exchanged for functionally equivalent parts as further described herein.

There is furthermore provided a nucleic acid sequence comprising the nucleic acid sequence of SEQ ID NO:5, or a functional equivalent thereof encoded by a nucleic acid sequence having 80% identity therewith, such as at least 85%, 90%, 91%, 92%, 93%, 94%, 97 % or 98% identity therewith.

Am open reading frame (ORF) for the Tyr1 is presented in SEQ ID NO: 15, and is also encompassed by the present disclosure. As mentioned herein, Tyr1 is a synthetic protein.

The present disclosure will now be illustrated and exemplified in the following experimental section, but it not intended to be limited thereto.

### EXPERIMENTAL SECTION

### Sequence listing:

**SEQ ID NO:7: Primer Tyr1_frw_KpnI**
   AGC GGT ACC GGT AAC AAG TAT AGA GTT AGA AAA AA
**SEQ ID NO:8: Primer Tyr1_rev_SacI**
   AAA GAG CTC TGA GGA ACG TTT TGA TTT TCT TA
**SEQ ID NO:9: Primer Tyr2_frw_KpnI**
   AAA GGT ACC GCG TGG CTG GTC GGC AAG
**SEQ ID NO:10: Primer Tyr2_rev_SacI**
   AGC GAG CTC GGC GGA CAC TAT GGC TAT TTC T
**SEQ ID NO:11: Primer Core-tyr2_frw_KpnI**
   AAA GGT ACC TCA AAT CCA AGG GAG AGT
**SEQ ID NO:12: Primer Core-tyr2_rev_SacI**
   AAA GAG CTC GAC CTT GAA GTA GGT CCG
**SEQ ID NO:13: Primer Tyr_IC_frw_HindIII**
   GAAAAGCTTCATATGGGTAACAAGTATAGAGTTAGAAAAAA
**SEQ ID NO:14: Primer Tyr_IC_rev_SgsI**
   AAA GGC GCG CCT TAT GAG GAA CGT TTT GAT TTT CTT A

### Material and Methods

### Strains and plasmids

Supporting table 1 shows a summary of the plasmids and strains used in this study. The plasmid pAIDA1¹⁵, was used for surface expression of the tyrosinases. This plasmid uses the AIDA-I surface expression system fused to a recombinant passenger protein flanked by an N-terminal His₆-detection tag and a C-terminal Myc detection tag (figure 1A). The strain *E*. *coli* 0: 17ΔOmpT²³, previously used for AIDA^{c} mediated surface expression⁸, was the host strain in all surface expression experiments. The tyrosinases used in this study are fused to the autotransporter AIDA, generating Tyr1-AIDA^{c} and Tyr2-AIDA^{c} Tyr1 (Supporting table 1) has some sequence similarity to the wild type *B. megaterium* tyrosinase while Tyr2 is identical to the *R. etli* wild type⁶. Both were PCR amplified from plasmids using the primers listed in supporting table 2 and ligated into the *Kpn*I and *Sac*I restriction sites in pAIDA1 giving the plasmids. In addition, a truncated "core" version of Tyr2 was created using homology modelling and cloned into pAIDA1, generating Tyr2_core-AIDA^{c}. Finally, Tyr1 was also ligated into the HindIII and SgsI(AscI) restriction sites of pAIDA1, creating an intracellular control (pTyr_IC) for expressed Tyr1.

The *E.coli* K12 0:17 strain can e.g. be purchased from SSI Diagnostica (Statens Serum Institut), Denmark, http://www.ssi.dk/ssidiagnostica. In *E.coli* K12 0:17ΔompT, the ompT gene has been deleted by conventional means.

**Supporting table 1: Strains and plasmids used in this study**

| **Strains and plasmids** | **Description** | **Source** |
|---|---|---|
| *Escherichia coli* | | |
| K12 0:17ΔompT | Δ*ompT* (*sup*⁺, F⁻) | 23 |
| DH5α | Standard gene cloning strain | |

| *Plasmids* | | |
|---|---|---|
| pTyr1 | Plasmid containing tyrosinase with sequence similarity to *Bacillus megaterium* tyrosinase | This study |
| pAIDA1 | AIDA-based surface display vector | 3 |
| pAIDA1-Tyr1 | Fused expression vector containing Tyr1 tyrosinase from pTyr1 | (SEQ ID NO:4) |
| pTrc99A | *Rhizobium etli* tyrosinase | 5 |
| pAIDA1-Tyr2_core | Fused expression vector containing *R.etli* core tyrosinase | This study |
| pAIDA1-Tyr2 | Fused expression vector containing R.etli tyrosinase | This study |
| pTyr_IC | Construct for intracellular expression of Tyr | This study |

**Supporting table 2: Primers used in this study**

| **Primer** | **Sequence** |
|---|---|
| Tyr1_frw_KpnI | AGC GGT ACC GGT AAC AAG TAT AGA GTT AGA AAA AA (SEQ ID NO:7) |
| Tyr1_rev_SacI | AAA GAG CTC TGA GGA ACG TTT TGA TTT TCT TA (SEQ ID NO:8) |
| Tyr2_frw_KpnI | AAA GGT ACC GCG TGG CTG GTC GGC AAG (SEQ ID NO:9) |
| Tyr2_rev_SacI | AGC GAG CTC GGC GGA CAC TAT GGC TAT TTC T (SEQ ID NO: 10) |
| Core-tyr2_frw_KpnI | AAA GGT ACC TCA AAT CCA AGG GAG AGT (SEQ ID NO:11) |
| Core-tyr2_rev_SacI | AAA GAG CTC GAC CTT GAA GTA GGT CCG (SEQ ID NO:12) |
| Tyr_IC_frw_HindIII | GAAAAGCTTCATATG GGTAACAAGTATAGAGTTAGAAAAAA (SEQ ID NO:13) |
| Tyr_IC_rev_SgsI | AAA GGC GCG CCT TAT GAG GAA CGT TTT GAT TTT CTT A (SEQ ID NO:14) |

### Features of the vector pAIDA-Tyrl (SEQ ID NO:4)

Features :
PlacUV5: [3683 : 3746 - CW]
Ori: [92 : 869 - CW]
Pcm : [1681 : 1800 - CW]
Cm : [1801 : 2457 - CW]
PlacI: [2473 : 2550 - CW]
LacI: [2551 : 3633 - CW]
HindIII: [3771 : 3775 - CW]
Signal peptide: [3779 : 3925 - CW]
His6: [3932 : 3949 - CW]
3C: [3950 : 3973 - CW]
TEV: [4874 : 4894 - CW]
Myc : [4895 : 4924 - CW]
Linker: [4931 : 5092 - CW]
AIDAc: [5102 : 6427 - CW]
Tyr1: [3980 : 4867 - CW]

### Cultivation media and growth conditions

All cell growth was performed using minimal salts medium as described previously 8 at pH 7.5 and 30°C in an orbital shaker incubator. The culture medium was supplemented with and 100 µg/mL chloramphenicol for plasmid selection. For solid medium, a 20X stock of minimal salts was prepared separately and added to 1.5% agar along with all of the above components.

Bioreactor cultures for design and enhancement of the surface reaction and melanin deposition were performed in a 10L bioreactor (Belach Bioteknik AB) with a working volume of 5L. The bioreactor medium was supplemented with 15 g/L glucose. Cells were grown at 30°C at a constant dissolved oxygen tension (DOT) of 40% air saturation and pH was kept at 7.5 through automatic titration with NH₄OH (25% w/v) and H₃PO₄ (10% w/v). When the optical density at 600 nm (OD600) reached 2, tyrosinase production was induced by addition of IPTG and CuSO₄ to a concentration of 200µM and 10µM, respectively. Melanin production was initiated by addition of sterile tyrosine solution to a concentration of 1 g/L as the culture entered the stationary phase due to glucose depletion under fully oxygenated conditions. The access to tyrosine was significantly improved by a dissolving protocol where a stock solution was prepared in water and titrated by KOH to pH 10.5.

### Cell harvest

Cells from bioreactor cultures were harvested through centrifugation (3000g, 15 min, 4°C) and the resulting cell pellet was dissolved in phosphate buffered saline (PBS, pH 7.0). Both the medium supernatant and the dissolved cell suspension were stored at 4°C.

### Analyses

### Cell growth

Cell growth was followed by monitoring OD600 and by measurement of cell dry weight (CDW). CDW was measured by withdrawing 5 ml cell suspension samples in triplicates into dried, preweighed glass tubes. Cells were pelleted by centrifugation (4500 rpm, 5 min) and the resulting cell pellet was dried over night at 105°C before weighing.

### Flow cytometry

Samples of cells and medium were taken during cultivation and diluted to an OD600 of approximately 1, mixed 50/50 with sterile glycerol solution (50% v/v) and frozen at -80°C for preservation, as described previously8. At the day of analysis, 50 µl of frozen samples were thawed and washed with 800 µl PBS. After centrifugation (10 minutes, 4500 rpm, 4°C) the supernatant was discarded and the remaining cell pellets were resuspended in 100 µl PBS containing antibody solution.

For detection surface expressed proteins 10 µg/mL THETM His Tag FITC antibody (Genscript) and 5 µg/mL anti-c-Myc SureLight® allophycocyanin antibody (Abcam) was used. Samples were incubated in room temperature for 60 minutes with end-over-end mixing. The solution was then centrifuged (10 minutes, 4500 rpm, 4°C) to pellet the cells, which were washed with 100 µl PBS followed by centrifugation (10 minutes, 4500 rpm, 4°C) and resuspended in 500 µl PBS. Surface expression levels were then evaluated by the fluorescence of the antibodies using a flow cytometer (Gallios, Beckman Coulter). The excitation wavelength was 488 nm for the His-tag and Alexa488-tag analysis and emission was detected at 525/40 nm. For the c-Myc-analysis 638 nm and 660/20 nm was used. 10,000 cells were recorded per sample.

### Protein isolation from subcellular compartments

Soluble, inner membrane and outer membrane proteins were isolated as previously described24. Centrifugation (2500 rpm, 15 minutes, 4°C) (J-6B Centrifuge, Beckman) was used to harvest 500 ml cultures four generations after induction. The pellet was washed using 140 mL 50 mM Tris-HCl (pH 7.5) and centrifuged under the same conditions as previously. The obtained pellet was stored overnight in 4°C. The following day, 1.75 g of cell pellet was dissolved in 11 mL 50 mM Tris-HCl (pH 7.5) and disintegrated in a French press high-pressure homogenizer (SLM Aminco). Non-disintegrated cells were removed by centrifugation (2000 g, 15 minutes, 4°C) (J-20 XP Centrifuge, Beckman). The supernatant was centrifuged (36000 g, 40 minutes, 4°C) to collect membrane bound proteins in the pellet. The resulting supernatant contains the soluble proteins and was collected and stored at -20°C until analysis. The remaining pellet was washed with 1.6 mL 50 mM Tris-HCl (pH 7.5) and centrifuged (36000 g, 40 minutes, 4°C). The pellet was dissolved in 12 mL 50 mM Tris-HCl with 0.1 % (v/v) sarcosyl (N-laurylsarcosine sodium salt solution, Fluka) and incubated on a shaking table for 1 hour at 4°C. The suspension was centrifuged (36000 g, 40 minutes, 4°C) and the supernatant containing the inner membrane proteins was collected and stored at -20°C until analysis. The remaining pellet containing the outer membrane proteins was dissolved in 7 mL 50 mM Tris-HCl with 5 mM EDTA and 1 % (v/v) Triton-X-100 (SigmaUltra, Sigma-Aldrich) and stored at -20°C until analysis.

### Western blot

Soluble, inner membrane and outer membrane protein fractions were thawed and loaded on a 10 % SDS-PAGE gel (Bis-Tris, NuPAGE, Invitrogen) and run at 180 V. The gel was blotted to a nitrocellulose membrane, blocked overnight with PBS containing 5 % milk powder. The next day the membrane was incubated for 60 minutes in PBST containing 10 g/L Bovine serum albumin (BSA) and either 0.5 µg/mL antibody (THETM c-Myc [HRP], GenScript) or rabbit antiserum against AIDAc. After washing in PBST, the membranes were developed as described previously 8, 15.

### Melanin formation

The formation of melanin was followed by measuring the absorbance at 400 nm (A400) of the supernatant from the CDW measurements, as previously described²⁵. In addition, melanin from 4 ml supernatant samples were precipitated by lowering the pH to 2 using 6M HCl followed by centrifugation (4500 rpm, 5 min). For extraction of cell-associated melanin, cells were treated over night with 1M KOH at 60°C. After overnight treatment, the alkaline solution was filtered (0.2 µm sterile cellulose filters, VWR). The pH of the solution was adjusted to 2, HCl, and centrifuged for 5 min 4500 g. The precipitate were washed twice with deionized water and dried over night at 40°C. The resulting precipitates were dried over-night at 105°C and analysed by Fourier transform infrared spectroscopy (FTIR) using a Perkin-Elmer Spectrum 2000 FTIR instrument (Norwalk, CT), The resulting spectra were compared to a reference sample of commercial synthetic melanin (Sigma Aldrich).

### API (Pharmaceutical substance) binding assay

*E. coli* cells producing melanin on the surface where assayed for affinity to the antimalarial drug chloroquine, which has previously been reported to adsorb to melanin 17. Cells and drugs dissolved in PBS buffer at pH 7.0 were used for all adsorption experiments. Cell solution (5g/L) and chloroquine solution were mixed in equal parts to a total volume of 1 ml in a 1,5 ml microcentrifuge tubes. Samples were incubated (25°C, 900 rpm) for 30 min in a microcentrifuge tube mixer (Thermomixer compact, Eppendorf) to avoid sedimentation. Cells were separated from the solution using a centrifuge (Heraus, Biofuge fresco) at 16060g for 10 min. 200 µl were pipetted from the top of the solution and added to an HPLC vial containing 800 µl PBS solution (pH 7.0). Chloroquine was varied in different concentrations in-between (35µM-2mM). All adsorption experiments were performed in triplicates. Samples were analysed using high performance liquid chromatography (Alliance, Waters 2695). A reversed-phase Novapak C18 column, 3.9x300mm, with 4µm particle size was used. The mobile phase was prepared by mixing a solution of 15:85 acetonitrile:MilliQ water, afterwards 1% trimethylamine was added and pH was adjusted to 2.8 with 89% H₃PO₄²⁶. The mobile phase was degassed and set to flow at 1 ml/min. The column effluent was monitored for 20 minutes at 343 nm using a Waters 2996 photodiode array detector.

### Visualization of melanin by whole-cell immunofluorescence microscopy

For detection of surface correlated melanin by flow cytometry, cells were incubated with 6D2 mouse anti-melanin IgM antibody (50µg/ml) over night at 4°C with end-over-end mixing. The solution was then centrifuged (10 minutes, 4500 rpm, 4°C), and the pellet was washed with 100 µl PBS and centrifuged again. The pellets were dissolved in 100µl PBS with 1,5µg/ml Alexa Fluor® 488 AffiniPure Donkey Anti-Mouse IgM, (Jacksson Immuno) and incubated (1 h, room temperature) covered in aluminum foil. After incubation, samples were centrifuged, washed two times, and resuspended in 500µl PBS for flow cytometry and 100µl PBS for visualization of melanin by whole-cell immunofluorescence microscopy. Finally, the fluorescence was quantified using flow cytometry, as described for the His6-antibody above.

For analysis by fluorescent microscopy the samples were treated with melanin specific antibodies as described above, redissolved in 100µl PBS and viewed at 1000X in an Olympus BX51 microscopy using a fluorescein filter.

### REFERENCES

1. Nakamura, C.E. & Whited, G.M. Metabolic engineering for the microbial production of 1,3-propanediol. Current Opinion in Biotechnology 14, 454-459 (2003).
2. Fujita, Y. et al. Direct and Efficient Production of Ethanol from Cellulosic Material with a Yeast Strain Displaying Cellulolytic Enzymes. Applied and Environmental Microbiology 68, 5136-5141 (2002).
3. Lerner, A.B. & Fitzpatrick, T.B. Biochemistry of melanin formation. Physiological Reviews 30, 91-126 (1950).
4. Karlsson, O. & Lindquist, N.G. Melanin affinity and its possible role in neurodegeneration. Journal of Neural Transmission 120, 1623-1630 (2013).
5. Shuster, V. & Fishman, A. Isolation, cloning and characterization of a tyrosinase with improved activity in organic solvents from Bacillus megaterium. Journal of Molecular Microbiology 17, 188-200 (2009).
6. Cabrera-Valladares, N. et al. Expression of the melA gene from Rhizobium etli CFN42 in Escherichia coli and characterization of the encoded tyrosinase. Enzyme and Microbial Technology 38, 772-779 (2006).
7. Gustavsson, M. in Biotechnology, Vol. PhD (Kungliga tekniska högskolan (KTH), 2013).
8. Gustavsson, M., Backlund, E. & Larsson, G. Optimisation of surface expression using the AIDA autotransporter. Microbial Cell Factories 10, 72 (2011).
9. Jose, J., Kramer, J., Klauser, T., Pohlner, J. & Meyer, T.F. Absence of periplasmic DsbA oxidoreductase facilitates export of cysteine-containing passenger proteins to the Escherichia coli cell surface via the Iga beta autotransporter pathway. Gene 178, 107-110 (1996).
10. Shokri, A., Sanden, A.M. & Larsson, G. Cell and process design for targeting of recombinant protein into the culture medium of Escherichia coli. Applied Microbiology and Biotechnology 60, 654-664 (2003).
11. Benz, I. & Schmidt, M.A. Cloning and expression of an adhesin (AIDA-I) involved in diffuse adherence of enteropathogenic Escherichia coli. Infection and Immunity 57, 1506-1511 (1989).
12. Leyton, D.L., Rossiter, A.E. & Henderson, I.R. From self sufficiency to dependence: mechanisms and factors important for autotransporter biogenesis. Nature Reviews Microbiology 10, 213-225 (2012).
13. Gustavsson, M., Muraleedharan, M.N. & Larsson, G. Surface expression of omega-transaminase in Escherichia coli. Applied Environmental Microbiology 80, 2293-2298 (2014).
14. Gustavsson, M. et al. Improved cell surface display of Salmonella enterica serovar Enteritidis antigens in Escherichia coli. Microbial Cell Factories 14, 47 (2015).
15. Jarmander, J., Gustavsson, M., Do, T.H., Samuelson, P. & Larsson, G. A dual tag system for facilitated detection of surface expressed proteins in Escherichia coli. Microbial Cell Factories 11, 118 (2012).
16. Fairhead, M. & Thony-Meyer, L. Role of the C-terminal extension in a bacterial tyrosinase. FEBS Journal 277, 2083-2095 (2010).
17. Lagunas-Munoz, V.H., Cabrera-Valladares, N., Bolivar, F., Gosset, G. & Martinez, A. Optimum melanin production using recombinant Escherichia coli. Journal of Applied Microbiology 101, 1002-1008 (2006).
18. Aghajanyan, A.E. et al. Isolation, purification and physicochemical characterization of watersoluble Bacillus thuringiensis melanin. Pigment Cell Research 18, 130-135 (2005).
19. Harki, E., Talou, T. & Dargent, R. Purification, characterisation and analysis of melanin extracted from Tuber melanosporum Vitt. Food Chemistry 58, 69-73 (1997).
20. Loos, R. et al. EU-wide monitoring survey on emerging polar organic contaminants in wastewater treatment plant effluents. Water Research 47, 6475-6487 (2013).
21. Lange, R. & Dietrich, D. Environmental risk assessment of pharmaceutical drug substances-conceptual considerations. Toxicology Letters 131, 97-104 (2002).
22. Larsson, B.S. Interaction Between Chemicals and Melanin. Pigment Cell Research 6, 127-133 (1993).
23. Olsson, M.O. & Isaksson, L.A. Analysis of rpsD mutations in Escherichia coli. III. Effects of rpsD mutations on expression of some ribosomal protein genes. Molecular Genetics and Genomics 169, 271-278 (1979).
24. Backlund, E. et al. Fedbatch design for periplasmic product retention in Escherichia coli. Journal of Biotechnology 135, 358-365 (2008).
25. Turick, C.E., Tisa, L.S. & Caccavo, F., Jr. Melanin production and use as a soluble electron shuttle for Fe(III) oxide reduction and as a terminal electron acceptor by Shewanella algae BrY. Applied Environmental Microbiology 68, 2436-2444 (2002).
26. Bell, D.J., Nyirongo, S.K., Molyneux, M.E., Winstanley, P.A. & Ward, S.A. Practical HPLC methods for the quantitative determination of common antimalarials in Africa. Journal of Chromatography B 847, 231-236 (2007).

## Claims

1. An expression vector for expressing a tyrosinase (Tyr1) to be presented on the surface of a bacterial cell, said expression vector comprising an expression cassette encoding a tyrosinase (Tyr1) as a passenger in a Type V secretion β-autotransporter system, wherein said tyrosinase (Tyr1) when expressed comprises an amino acid sequence of SEQ ID NO:1.

2. The expression vector according to claim 1, wherein said tyrosinase (Tyr1) is encoded by a nucleic acid sequence comprising SEQ ID NO:2, or a sequence having at least 80%, such as 85, 90 or 95% identity with SEQ ID NO:2.

3. The expression vector according to any one of the preceding claims, wherein said expression cassette encodes a protein which is the tyrosinase (Tyr1) as a passenger in a Type V secretion β-autotransporter system AIDA (Adhesin Involved in Diffuse Adherence), such as Type V β-autotransporter system AIDA-I (SEQ ID NO:3), or encodes a protein having at least 95% identity with SEQ ID NO:3.

4. The expression vector according to claim 3, wherein the protein is encoded by a nucleic acid sequence comprising SEQ ID NO:5, or a sequence having at least 80% identity, such as 85%, 90% or 95% identity with SEQ ID NO:5.

5. The expression vector according to any of claims 1-4, wherein said expression vector is pAIDA1-Tyr1 (SEQ ID NO:4), or an expression vector having at least 80% sequence identity therewith, such as 85%, 90% or 95% identity with the sequence of SEQ ID NO:4.

6. A host cell comprising an expression vector according to any one of claims 1-5, such as an *E.coli* cell, such as *E.coli* K12 strain, preferably *E.coli* 0:17K12ΔOmpT.

7. A method for producing one or more bacterial cell(s) at least partially coated with a chemical entity-adsorbing composition, said method comprising the steps of:
a) selecting and culturing one or more bacterial cell(s) comprising an expression vector according to any one of claims 1-5 or a host cell according to claim 6.
b) inducing the expression of tyrosinase (Tyr1) from said expression vector to allow presentation of said tyrosinase (Tyr1) on the surface of said one or more bacterial cell(s),
c) adding a suitable amount of tyrosine, or a functional equivalent thereof, to said one or more bacterial cell(s) to allow the formation of said chemical entity-adsorbing composition at least partially on the surface of said one or more bacterial cell(s), and thereafter
d) retrieving the cells from step c).

8. The method according to claim 7, wherein the culturing of the cells is performed at a pH of about 7-8, such as at about pH 7.5, and optionally wherein the induction in step b) is performed by adding IPTG or lactose to the bacterial cell culture, such as in an amount of about 200 µM IPTG per induction.

9. The method according to any one of claims 7-8, wherein expression of the tyrosinase (Tyr1) is allowed to continue during a minimum of two cell generations after the induction in step b), such as for 2, 3, 4, 5, or 6 generations.

10. The method according to any one of claims 7-9, wherein tyrosine is in the form of a powder composition or contained in an aqueous basic solution having a pH of about 9-12, such as about pH 10.

11. The method according to any one of claims 7-10, wherein the chemical entity-adsorbing composition comprises melanin, and/or a functional equivalent thereof.

12. The method according to any one of claims 7-11, wherein the chemical entity comprises a pharmaceutical substance, or one or more degradation product(s) thereof, or one or more heavy metal compound(s).

13. One or more bacterial cell(s) at least partially coated with a chemical entity-adsorbing composition obtainable by the method according to any one of claims 7-12.

14. Use of one or more bacterial cell(s) at least partially coated with a chemical entity-adsorbing composition according to claim 13, for the removal of one or more chemical entities from water, such as wastewater.

15. Use according to claim 14, wherein said one or more bacterial cell(s) are suspended in a solution or present in a biofilm, optionally wherein said biofilm is present on a solid support, such as a stationary solid support or a moving solid support.

16. A device, such as a MBR (Membrane Bioreactor), comprising one or more bacterial cell(s) according to claim 13 suspended in a solution.

17. A device, such as a MBBR (Moving bed biofilm reactor) comprising one or more bacterial cell(s) according to claim 13 present in a biofilm on a solid support.

18. A method for the removal of one or more chemical entities from water, such as wastewater, comprising the steps of:
a) Providing one or more bacterial ccll(s) of claim 13, or a biofilm comprising said one or more bacterial cell(s),
b) Allowing water to pass through the one or more bacterial cell(s), or said biofilm comprising said one or more bacterial cell(s), thereby adsorbing any chemical entities present in the water thereto,
c) Removing any adsorbed chemical entities from the one or more bacterial cell(s), or biofilm comprising said one or more bacterial cell(s), by rinsing with an aqueous composition having an acidic pH, to thereafter allow for reuse of the one or more bacterial cell(s), or biofilm comprising said one or more bacterial cell(s), and thereafter
d) Optionally repeating steps b) and c).

## Patentansprüche

1. Expressionsvektor zum Exprimieren einer Tyrosinase (Tyr1), die auf der Oberfläche einer Bakterienzelle präsentiert werden soll, wobei der Expressionsvektor eine Expressionskassette umfasst, die für eine Tyrosinase (Tyr1) als ein Passagier in einem Typ-V-Sekretion-β-Autotransportersystem codiert, wobei die Tyrosinase (Tyr1), wenn sie exprimiert wird, eine Aminosäuresequenz von SEQ ID NO:1 umfasst.

2. Expressionsvektor nach Anspruch 1, wobei die Tyrosinase (Tyr1) durch eine Nukleinsäuresequenz, die SEQ ID NO:2 umfasst, oder eine Sequenz, die mindestens 80 %, wie 85, 90 oder 95 % Identität mit SEQ ID NO:2 aufweist, codiert wird.

3. Expressionsvektor nach einem der vorstehenden Ansprüche, wobei die Expressionskassette für ein Protein codiert, das die Tyrosinase (Tyr1) als ein Passagier in einem Typ-V-Sekretion-β-Autotransportersystem AIDA (Adhesin Involved in Diffuse Adherence, an diffuser Adhäsion beteiligtes Adhäsin) ist, wie Typ-V-β-Autotransportersystem AIDA-I (SEQ ID NO:3), oder für ein Protein codiert, das mindestens 95 % Identität mit SEQ ID NO:3 aufweist.

4. Expressionsvektor nach Anspruch 3, wobei das Protein von einer Nukleinsäuresequenz, die SEQ ID NO:5 umfasst, oder einer Sequenz, die mindestens 80 % Identität, wie 85 %, 90 % oder 95 % Identität mit SEQ ID NO:5 aufweist, codiert wird.

5. Expressionsvektor nach einem der Ansprüche 1-4, wobei der Expressionsvektor pAIDA1-Tyr1 (SEQ ID NO:4) oder ein Expressionsvektor, der mindestens 80 % Sequenzidentität damit aufweist, wie 85 %, 90 % oder 95 % Identität mit der Sequenz von SEQ ID NO:4, ist.

6. Wirtszelle, umfassend einen Expressionsvektor nach einem der Ansprüche 1 bis 5, wie eine *E.coli-Zelle,* wie *E.coli* K12-Stamm, vorzugsweise *E.coli* 0:17K12ΔOmpT.

7. Verfahren zum Herstellen einer oder mehrerer Bakterienzelle(n), die mindestens teilweise mit einer Zusammensetzung beschichtet sind, die chemische Entität adsorbiert, wobei das Verfahren die folgenden Schritte umfasst:
a) Auswählen und Kultivieren einer oder mehrerer Bakterienzelle(n), die einen Expressionsvektor nach einem der Ansprüche 1 bis 5 umfassen, oder eine Wirtszelle nach Anspruch 6.
b) Induzieren der Expression von Tyrosinase (Tyr1) aus dem Expressionsvektor, um eine Präsentation der Tyrosinase (Tyr1) auf der Oberfläche der einen oder mehreren Bakterienzelle(n) zu ermöglichen,
c) Zugeben einer geeigneten Menge an Tyrosin oder eines funktionellen Äquivalents davon zu der einen oder den mehreren Bakterienzelle(n), um die Bildung der Zusammensetzung, die eine chemische Entität adsorbiert, mindestens teilweise auf der Oberfläche der einen oder der mehreren Bakterienzelle(n) zu ermöglichen, und danach
d) Rückgewinnen der Zellen aus Schritt c).

8. Verfahren nach Anspruch 7, wobei das Kultivieren der Zellen bei einem pH-Wert von etwa 7 bis 8, wie bei etwa pH-Wert 7,5, durchgeführt wird und wobei gegebenenfalls die Induktion in Schritt b) durch Zugeben von IPTG oder Lactose zu der Bakterienzellkultur, wie in einer Menge von etwa 200 µM IPTG pro Induktion, durchgeführt wird.

9. Verfahren nach einem der Ansprüche 7 bis 8, wobei eine Expression der Tyrosinase (Tyr1) während eines Minimums von zwei Zellgenerationen nach der Induktion in Schritt b), wie für 2, 3, 4, 5 oder 6 Generationen, fortgesetzt werden kann.

10. Verfahren nach einem der Ansprüche 7 bis 9, wobei Tyrosin in Form einer Pulverzusammensetzung vorliegt oder in einer wässrigen basischen Lösung mit einem pH-Wert von etwa 9 bis 12, wie etwa pH-Wert 10, enthalten ist.

11. Verfahren nach einem der Ansprüche 7 bis 10, wobei die Zusammensetzung, die eine chemische Entität adsorbiert, Melanin und/oder ein funktionelles Äquivalent davon umfasst.

12. Verfahren nach einem der Ansprüche 7 bis 11, wobei die chemische Entität eine pharmazeutische Substanz oder ein oder mehrere Abbauprodukt(e) davon oder eine oder mehrere Schwermetallverbindung(en) umfasst.

13. Eine oder mehrere Bakterienzelle(n), die mindestens teilweise mit einer Zusammensetzung, die eine chemische Entität adsorbiert, beschichtet sind, die durch das Verfahren nach einem der Ansprüche 7 bis 12 erhältlich ist.

14. Verwendung einer oder mehrerer Bakterienzelle(n), die mindestens teilweise mit einer Zusammensetzung, die eine chemische Entität adsorbiert, nach Anspruch 13 beschichtet sind, zur Entfernung von einer oder mehreren chemischen Entitäten aus Wasser, wie Abwasser.

15. Verwendung nach Anspruch 14, wobei die eine oder mehreren Bakterienzelle(n) in einer Lösung suspendiert sind oder in einem Biofilm vorhanden sind, wobei der Biofilm gegebenenfalls auf einem festen Träger, wie einem stationären festen Träger oder einem sich bewegenden festen Träger, vorhanden ist.

16. Vorrichtung, wie ein MBR (Membranbioreaktor), umfassend eine oder mehrere Bakterienzelle(n) nach Anspruch 13, die in einer Lösung suspendiert sind.

17. Vorrichtung, wie ein MBBR (Bewegtbett-Biofilmreaktor), umfassend eine oder mehrere Bakterienzelle(n) nach Anspruch 13, die in einem Biofilm auf einem festen Träger vorhanden sind.

18. Verfahren zur Entfernung von einer oder mehreren chemischen Entitäten aus Wasser, wie Abwasser, umfassend die folgenden Schritte:
a) Bereitstellen einer oder mehrerer Bakterienzelle(n) nach Anspruch 13 oder eines Biofilms, der die eine oder mehreren Bakterienzelle(n) umfasst,
b) Durchlaufenlassen von Wasser durch die eine oder mehreren Bakterienzelle(n) oder den Biofilm, der die eine oder mehreren Bakterienzelle(n) umfasst, wodurch sämtliche chemischen Entitäten, die in dem Wasser vorhanden sind, daran adsorbiert werden,
c) Entfernen sämtlicher adsorbierter chemischer Entitäten aus der einen oder den mehreren Bakterienzelle(n) oder dem Biofilm, der die eine oder mehreren Bakterienzelle(n) umfasst, durch Spülen mit einer wässrigen Zusammensetzung mit einem sauren pH-Wert, um danach die Wiederverwendung der einen oder mehreren Bakterienzelle(n) oder des Biofilms, der die eine oder mehreren Bakterienzelle(n) umfasst, zu ermöglichen, und danach
d) gegebenenfalls Wiederholen von Schritten b) und c).

## Revendications

1. Vecteur d'expression pour exprimer une tyrosinase (Tyr1) à présenter sur la surface d'une cellule bactérienne, ledit vecteur d'expression comprenant une cassette d'expression codant pour une tyrosinase (Tyr1) en tant que passager dans un système d'autotransporteur β de sécrétion de Type V, dans lequel ladite tyrosinase (Tyr1) lorsqu'elle est exprimée comprend une séquence d'acides aminés de SEQ ID NO:1.

2. Vecteur d'expression selon la revendication 1, dans lequel ladite tyrosinase (Tyr1) est codée par une séquence d'acides nucléiques comprenant SEQ ID NO:2, ou une séquence ayant une identité d'au moins 80 %, telle que 85, 90 ou 95 % avec SEQ ID NO:2.

3. Vecteur d'expression selon l'une quelconque des revendications précédentes, dans lequel ladite cassette d'expression code pour une protéine qui est la tyrosinase (Tyr1) en tant que passager dans un système d'autotransporteur β de sécrétion de Type V AIDA (adhésine impliquée dans l'adhérence diffuse), tel qu'un système d'autotransporteur β de Type V AIDA-I (SEQ ID NO:3), ou code pour une protéine ayant au moins 95 % d'identité avec SEQ ID NO:3.

4. Vecteur d'expression selon la revendication 3, dans lequel la protéine est codée par une séquence d'acides nucléiques comprenant SEQ ID NO:5, ou une séquence ayant au moins 80 % d'identité, telle que 85 %, 90 % ou 95 % d'identité avec SEQ ID NO:5.

5. Vecteur d'expression selon l'une quelconque des revendications 1 à 4, dans lequel ledit vecteur d'expression est pAIDA1-Tyr1 (SEQ ID NO:4), ou un vecteur d'expression ayant une identité de séquence d'au moins 80 % avec celle-ci, telle que 85 %, 90 % ou 95 % d'identité avec la séquence de SEQ ID NO:4.

6. Cellule hôte comprenant un vecteur d'expression selon l'une quelconque des revendications 1 à 5, telle qu'une cellule d'*E.coli,* telle que la souche K12 d'*E.coli,* de préférence *E.coli* 0:17K12ΔOmpT.

7. Procédé de production d'une ou plusieurs cellule(s) bactérienne(s) au moins partiellement revêtue(s) d'une composition d'adsorption d'entité chimique, ledit procédé comprenant les étapes consistant à :
a) sélectionner et cultiver une ou plusieurs cellule(s) bactérienne(s) comprenant un vecteur d'expression selon l'une quelconque des revendications 1 à 5 ou une cellule hôte selon la revendication 6.
b) induire l'expression de tyrosinase (Tyr1) à partir dudit vecteur d'expression pour permettre la présentation de ladite tyrosinase (Tyr1) sur la surface de ladite ou desdites cellule(s) bactérienne(s),
c) ajouter une quantité appropriée de tyrosine, ou d'un équivalent fonctionnel de celle-ci, à ladite ou auxdites cellule(s) bactérienne(s) pour permettre la formation de ladite composition d'adsorption d'entité chimique au moins partiellement sur la surface de ladite ou desdites cellule(s) bactérienne(s), et par la suite
d) récupérer les cellules provenant de l'étape c).

8. Procédé selon la revendication 7, dans lequel la culture des cellules est mise en œuvre à un pH d'environ 7 à 8, telle qu'à environ pH 7,5, et facultativement dans lequel l'induction à l'étape b) est mise en œuvre en ajoutant de l'IPTG ou du lactose à la culture de cellules bactériennes, tel qu'en une quantité d'environ 200 µM d'IPTG par induction.

9. Procédé selon l'une quelconque des revendications 7 ou 8, dans lequel l'expression de la tyrosinase (Tyr1) est autorisée à se poursuivre pendant un minimum de deux générations de cellules après l'induction à l'étape b), telle que pendant 2, 3, 4, 5, ou 6 générations.

10. Procédé selon l'une quelconque des revendications 7 à 9, dans lequel la tyrosine est sous la forme d'une composition en poudre ou contenue dans une solution aqueuse basique ayant un pH d'environ 9 à 12, tel qu'environ pH 10.

11. Procédé selon l'une quelconque des revendications 7 à 10, dans lequel la composition d'adsorption d'entité chimique comprend de la mélanine, et/ou un équivalent fonctionnel de celle-ci.

12. Procédé selon l'une quelconque des revendications 7 à 11, dans lequel l'entité chimique comprend une substance pharmaceutique, ou un ou plusieurs produit(s) de dégradation de celle-ci, ou un ou plusieurs composé(s) de métaux lourds.

13. Cellule(s) bactérienne(s) au moins partiellement revêtue(s) d'une composition d'adsorption d'entité chimique pouvant être obtenue(s) par le procédé selon l'une quelconque des revendications 7 à 12.

14. Utilisation d'une ou plusieurs cellule(s) bactérienne(s) au moins partiellement revêtue(s) d'une composition d'adsorption d'entité chimique selon la revendication 13, pour l'élimination d'une ou plusieurs entités chimiques d'une eau, telle que des eaux usées.

15. Utilisation selon la revendication 14, dans laquelle ladite ou lesdites cellule(s) bactérienne(s) sont en suspension dans une solution ou présente(s) dans un biofilm, facultativement dans laquelle ledit biofilm est présent sur un support solide, tel qu'un support solide stationnaire ou un support solide mobile.

16. Dispositif, tel qu'un MBR (bioréacteur à membrane), comprenant une ou plusieurs cellule(s) bactérienne(s) selon la revendication 13 en suspension dans une solution.

17. Dispositif, tel qu'un MBBR (réacteur à biofilm à lit mobile) comprenant une ou plusieurs cellule(s) bactérienne(s) selon la revendication 13 présente(s) dans un film biologique sur un support solide.

18. Procédé d'élimination d'une ou plusieurs entités chimiques d'une eau, telle que des eaux usées, comprenant les étapes consistant à :
a) fournir une ou plusieurs cellule(s) bactérienne(s) selon la revendication 13, ou un biofilm comprenant ladite ou lesdites cellule(s) bactérienne(s),
b) laisser passer l'eau à travers la ou les cellule(s) bactérienne(s), ou ledit biofilm comprenant ladite ou lesdites cellule(s) bactérienne(s), ce qui y adsorbe n'importe quelles entités chimiques présentes dans l'eau,
c) éliminer n'importe quelles entités chimiques adsorbées de la ou des cellule(s) bactérienne(s), ou du film biologique comprenant ladite ou lesdites cellule(s) bactérienne(s), en rinçant avec une composition aqueuse ayant un pH acide, pour permettre par la suite la réutilisation de la ou des cellule(s) bactérienne(s), ou du film biologique comprenant ladite ou lesdites cellule(s) bactérienne(s), et par la suite
d) facultativement répéter les étapes b) et c).


### SEQUENCE LISTING

<110> Ragn-Sells AB
<120> Improved means for water purification
<130> P10954SE00_69232
<160> 15
<170> PatentIn version 3.5
<210> 1
   <211> 296
   <212> PRT
   <213> Artificial sequence
<220>
   <223> Tyr1, synthetic tyrosinase
<400> 1
   Gly Asn Lys Tyr Arg Val Arg Lys Asn Val Leu His Leu Thr Asp Thr
   1               5                   10                  15
   Glu Lys Arg Asp Phe Val Arg Thr Val Leu Ile Leu Lys Glu Lys Gly
               20                  25                  30
   Ile Tyr Asp Arg Tyr Ile Ala Trp His Gly Ala Ala Gly Lys Phe His
           35                  40                  45
   Thr Pro Pro Gly Ser Asp Arg Asn Ala Ala His Met Ser Ser Ala Phe
       50                  55                  60
   Leu Pro Trp His Arg Glu Tyr Leu Leu Arg Phe Glu Arg Asp Leu Gln
   65                  70                  75                  80
   Ser Ile Asn Pro Glu Val Thr Leu Pro Tyr Trp Glu Trp Glu Thr Asp
                   85                  90                  95
   Ala Gln Met Gln Asp Pro Ser Gln Ser Gln Ile Trp Ser Ala Asp Phe
               100                 105                 110
   Met Gly Gly Asn Gly Asn Pro Ile Lys Asp Phe Ile Val Asp Thr Gly
           115                 120                 125
   Pro Phe Ala Ala Gly Arg Trp Thr Thr Ile Asp Glu Gln Gly Asn Pro
       130                 135                 140
   Ser Gly Gly Leu Lys Arg Asn Phe Gly Ala Thr Lys Glu Ala Pro Thr
   145                 150                 155                 160
   Leu Pro Thr Arg Asp Asp Val Leu Asn Ala Leu Lys Ile Thr Gln Tyr
                       165                 170                 175
   Asp Thr Pro Pro Trp Asp Met Thr Ser Gln Asn Ser Phe Arg Asn Gln
               180                 185                 190
   Leu Glu Gly Phe Ile Asn Gly Pro Gln Leu His Asn Arg Val His Arg
           195                 200                 205
   Trp Val Gly Gly Gln Met Gly Val Val Pro Thr Ala Pro Asn Asp Pro
       210                 215                 220
   Val Phe Phe Leu His His Ala Asn Val Asp Arg Ile Trp Ala Val Trp
   225                 230                 235                 240
   Gln Ile Ile His Arg Asn Gln Asn Tyr Gln Pro Met Lys Asn Gly Pro
                   245                 250                 255
   Phe Gly Gln Asn Phe Arg Asp Pro Met Tyr Pro Trp Asn Thr Thr Pro
               260                 265                 270
   Glu Asp Val Met Asn His Arg Lys Leu Gly Tyr Val Tyr Asp Ile Glu
           275                 280                 285
   Leu Arg Lys Ser Lys Arg Ser Ser
       290                 295
<210> 2
   <211> 888
   <212> DNA
   <213> Artificial sequence
<220>
   <223> ORF for Tyr1 of SEQ ID NO:1
<400> 2
   ggtaacaagt atagagttag aaaaaacgta ttacatctta ccgacacgga aaaaagagat      60
   tttgttcgta ccgtgctaat actaaaggaa aaagggatat atgaccgcta tatagcctgg     120
   catggtgcag caggtaaatt tcatactcct ccgggcagcg atcgaaatgc agcacatatg     180
   agttctgctt ttttaccgtg gcatcgtgaa taccttttac gattcgaacg tgaccttcag     240
   tcaatcaatc cagaagtaac ccttccttat tgggaatggg aaacggacgc acagatgcag     300
   gatccctcac aatcacaaat ttggagtgca gattttatgg gaggaaacgg aaatcccata     360
   aaagatttta tcgtcgatac cgggccattt gcagctgggc gctggacgac gatcgatgaa     420
   caaggaaatc cttccggagg gctaaaacgt aattttggag caacgaaaga ggcacctaca     480
   ctccctactc gagatgatgt cctcaatgct ttaaaaataa ctcagtatga tacgccgcct     540
   tgggatatga ccagccaaaa cagctttcgt aatcagcttg aaggatttat taacgggcca     600
   cagcttcaca atcgcgtaca ccgttgggtt ggcggacaga tgggcgttgt gcctactgct     660
   ccgaatgatc ctgtcttctt tttacaccac gcaaatgtgg atcgtatttg ggctgtatgg     720
   caaattattc atcgtaatca aaactatcag ccgatgaaaa acgggccatt tggtcaaaac     780
   tttagagatc cgatgtaccc ttggaataca acccctgaag acgttatgaa ccatcgaaag     840
   cttgggtacg tatacgatat agaattaaga aaatcaaaac gttcctca                  888
<210> 3
   <211> 881
   <212> PRT
   <213> Artificial sequence
<220>
   <223> Protein expressed from the vector pAIDA-Tyrl
<400> 3
   Met Asn Lys Ala Tyr Ser Ile Ile Trp Ser His Ser Arg Gln Ala Trp
   1               5                   10                  15
   Ile Val Ala Ser Glu Leu Ala Arg Gly His Gly Phe Val Leu Ala Lys
               20                  25                  30
   Asn Thr Leu Leu Val Leu Ala Val Val Ser Thr Ile Gly Asn Ala Phe
           35                  40                  45
   Ala Val Asp His His His His His His Leu Glu Ala Leu Phe Gln Gly
       50                  55                  60
   Pro Gly Thr Gly Asn Lys Tyr Arg Val Arg Lys Asn Val Leu His Leu
   65                  70                  75                  80
   Thr Asp Thr Glu Lys Arg Asp Phe Val Arg Thr Val Leu Ile Leu Lys
                   85                  90                  95
   Glu Lys Gly Ile Tyr Asp Arg Tyr Ile Ala Trp His Gly Ala Ala Gly
               100                 105                 110
   Lys Phe His Thr Pro Pro Gly Ser Asp Arg Asn Ala Ala His Met Ser
           115                 120                 125
   Ser Ala Phe Leu Pro Trp His Arg Glu Tyr Leu Leu Arg Phe Glu Arg
       130                 135                 140
   Asp Leu Gln Ser Ile Asn Pro Glu Val Thr Leu Pro Tyr Trp Glu Trp
   145                 150                 155                 160
   Glu Thr Asp Ala Gln Met Gln Asp Pro Ser Gln Ser Gln Ile Trp Ser
                   165                 170                 175
   Ala Asp Phe Met Gly Gly Asn Gly Asn Pro Ile Lys Asp Phe Ile Val
               180                 185                 190
   Asp Thr Gly Pro Phe Ala Ala Gly Arg Trp Thr Thr Ile Asp Glu Gln
           195                 200                 205
   Gly Asn Pro Ser Gly Gly Leu Lys Arg Asn Phe Gly Ala Thr Lys Glu
       210                 215                 220
   Ala Pro Thr Leu Pro Thr Arg Asp Asp Val Leu Asn Ala Leu Lys Ile
   225                 230                 235                 240
   Thr Gln Tyr Asp Thr Pro Pro Trp Asp Met Thr Ser Gln Asn Ser Phe
                   245                 250                 255
   Arg Asn Gln Leu Glu Gly Phe Ile Asn Gly Pro Gln Leu His Asn Arg
               260                 265                 270
   Val His Arg Trp Val Gly Gly Gln Met Gly Val Val Pro Thr Ala Pro
           275                 280                 285
   Asn Asp Pro Val Phe Phe Leu His His Ala Asn Val Asp Arg Ile Trp
       290                 295                 300
   Ala Val Trp Gln Ile Ile His Arg Asn Gln Asn Tyr Gln Pro Met Lys
   305                 310                 315                 320
   Asn Gly Pro Phe Gly Gln Asn Phe Arg Asp Pro Met Tyr Pro Trp Asn
                   325                 330                 335
   Thr Thr Pro Glu Asp Val Met Asn His Arg Lys Leu Gly Tyr Val Tyr
               340                 345                 350
   Asp Ile Glu Leu Arg Lys Ser Lys Arg Ser Ser Glu Leu Glu Asn Leu
           355                 360                 365
   Tyr Phe Gln Gly Glu Gln Lys Leu Ile Ser Glu Glu Asp Leu Ser Arg
       370                 375                 380
   Val Asn Asn Asn Gly Ser Ile Val Ile Asn Asn Ser Ile Ile Asn Gly
   385                 390                 395                 400
   Asn Ile Thr Asn Asp Ala Asp Leu Ser Phe Gly Thr Ala Lys Leu Leu
                   405                 410                 415
   Ser Ala Thr Val Asn Gly Ser Leu Val Asn Asn Lys Asn Ile Ile Leu
               420                 425                 430
   Asn Pro Thr Lys Glu Ser Ala Ala Ala Ile Gly Asn Thr Leu Thr Val
           435                 440                 445
   Ser Asn Tyr Thr Gly Thr Pro Gly Ser Val Ile Ser Leu Gly Gly Val
       450                 455                 460
   Leu Glu Gly Asp Asn Ser Leu Thr Asp Arg Leu Val Val Lys Gly Asn
   465                 470                 475                 480
   Thr Ser Gly Gln Ser Asp Ile Val Tyr Val Asn Glu Asp Gly Ser Gly
                   485                 490                 495
   Gly Gln Thr Arg Asp Gly Ile Asn Ile Ile Ser Val Glu Gly Asn Ser
               500                 505                 510
   Asp Ala Glu Phe Ser Leu Lys Asn Arg Val Val Ala Gly Ala Tyr Asp
           515                 520                 525
   Tyr Thr Leu Gln Lys Gly Asn Glu Ser Gly Thr Asp Asn Lys Gly Trp
       530                 535                 540
   Tyr Leu Thr Ser His Leu Pro Thr Ser Asp Thr Arg Gln Tyr Arg Pro
   545                 550                 555                 560
   Glu Asn Gly Ser Tyr Ala Thr Asn Met Ala Leu Ala Asn Ser Leu Phe
                   565                 570                 575
   Leu Met Asp Leu Asn Glu Arg Lys Gln Phe Arg Ala Met Ser Asp Asn
               580                 585                 590
   Thr Gln Pro Glu Ser Ala Ser Val Trp Met Lys Ile Thr Gly Gly Ile
           595                 600                 605
   Ser Ser Gly Lys Leu Asn Asp Gly Gln Asn Lys Thr Thr Thr Asn Gln
       610                 615                 620
   Phe Ile Asn Gln Leu Gly Gly Asp Ile Tyr Lys Phe His Ala Glu Gln
   625                 630                 635                 640
   Leu Gly Asp Phe Thr Leu Gly Ile Met Gly Gly Tyr Ala Asn Ala Lys
                   645                 650                 655
   Gly Lys Thr Ile Asn Tyr Thr Ser Asn Lys Ala Ala Arg Asn Thr Leu
               660                 665                 670
   Asp Gly Tyr Ser Val Gly Val Tyr Gly Thr Trp Tyr Gln Asn Gly Glu
   675 680 685
   Asn Ala Thr Gly Leu Phe Ala Glu Thr Trp Met Gln Tyr Asn Trp Phe
   690 695 700
   Asn Ala Ser Val Lys Gly Asp Gly Leu Glu Glu Glu Lys Tyr Asn Leu
   705 710 715 720
   Asn Gly Leu Thr Ala Ser Ala Gly Gly Gly Tyr Asn Leu Asn Val His
   725 730 735
   Thr Trp Thr Ser Pro Glu Gly Ile Thr Gly Glu Phe Trp Leu Gln Pro
   740 745 750
   His Leu Gln Ala Val Trp Met Gly Val Thr Pro Asp Thr His Gln Glu
   755 760 765
   Asp Asn Gly Thr Val Val Gln Gly Ala Gly Lys Asn Asn Ile Gln Thr
   770 775 780
   Lys Ala Gly Ile Arg Ala Ser Trp Lys Val Lys Ser Thr Leu Asp Lys
   785 790 795 800
   Asp Thr Gly Arg Arg Phe Arg Pro Tyr Ile Glu Ala Asn Trp Ile His
   805 810 815
   Asn Thr His Glu Phe Gly Val Lys Met Ser Asp Asp Ser Gln Leu Leu
   820 825 830
   Ser Gly Ser Arg Asn Gln Gly Glu Ile Lys Thr Gly Ile Glu Gly Val
   835 840 845
   Ile Thr Gln Asn Leu Ser Val Asn Gly Gly Val Ala Tyr Gln Ala Gly
   850 855 860
   Gly His Gly Ser Asn Ala Ile Ser Gly Ala Leu Gly Ile Lys Tyr Ser
   865 870 875 880
   Phe
<210> 4
   <211> 6433
   <212> DNA
   <213> Artificial sequence
<220>
   <223> Expression vector pAIDA1-Tyr1
<400> 4
   tggcacgcgg cgcgcccctt ggtgcgcaaa ctattaactg gcgaactact tactctagct      60
   tcccggcaac aattaataga ctggatggag gcggataaag ttgcaggacc acttctgcgc     120
   tcggcccttc cggctggctg gtttattgct gataaatctg gagccggtga gcgtgggtct     180
   cgcggtatca ttgcagcact ggggccagat ggtaagccct cccgtatcgt agttatctac     240
   acgacgggga gtcaggcaac tatggatgaa cgaaatagac agatcgctga gataggtgcc     300
   tcactgatta agcattggta actgtcagac caagtttact catatatact ttagattgat     360
   ttaaaacttc atttttaatt taaaaggatc taggtgaaga tcctttttga taatctcatg     420
   accaaaatcc cttaacgtga gttttcgttc cactgagcgt cagacccctt aataagatga     480
   tcttcttgag atcgttttgg tctgcgcgta atctcttgct ctgaaaacga aaaaaccgcc     540
   ttgcagggcg gtttttcgaa ggttctctga gctaccaact ctttgaaccg aggtaactgg     600
   cttggaggag cgcagtcacc aaaacttgtc ctttcagttt agccttaacc ggcgcatgac     660
   ttcaagacta actcctctaa atcaattacc agtggctgct gccagtggtg cttttgcatg     720
   tctttccggg ttggactcaa gacgatagtt accggataag gcgcagcggt cggactgaac     780
   ggggggttcg tgcatacagt ccagcttgga gcgaactgcc tacccggaac tgagtgtcag     840
   gcgtggaatg agacaaacgc ggccataaca gcggaatgac accggtaaac cgaaaggcag     900
   gaacaggaga gcgcacgagg gagccgccag ggggaaacgc ctggtatctt tatagtcctg      960
   tcgggtttcg ccaccactga tttgagcgtc agatttcgtg atgcttgtca ggggggcgga     1020
   gcctatggaa aaacggcttt gccgcggccc tctcacttcc ctgttaagta tcttcctggc    1080
   atcttccagg aaatctccgc cccgttcgta agccatttcc gctcgccgca gtcgaacgac    1140
   cgagcgtagc gagtcagtga gcgaggaagc ggaatatatc ctgtatcaca tattctgctg    1200
   acgcaccggt gcagcctttt ttctcctgcc acatgaagca cttcactgac accctcatca    1260
   gtgccaacat agtaagccag tatacactcc gctagcgctg aggtctgcct cgtgaagaag    1320
   gtgttgctga ctcataccag gcctgaatcg ccccatcatc cagccagaaa gtgagggagc    1380
   cacggttgat gagagctttg ttgtaggtgg accagttggt gattttgaac ttttgctttg    1440
   ccacggaacg gtctgcgttg tcgggaagat gcgtgatctg atccttcaac tcagcaaaag    1500
   ttcgatttat tcaacaaagc cacgttgtgt ctcaaaatct ctgatgttac attgcacaag    1560
   ataaaaatat atcatcatga acaataaaac tgtctgctta cataaacagt aatacaaggg    1620
   gtgttatgag ccatattcaa cgggaaacgt cttgctcgag tatccgctca tgagattatc    1680
   aaaaaggatc ttcacctaga tccttttgta agaggttcca actttcacca taatgaaata    1740
   agatcactac cgggcgtatt ttttgagtta tcgagatttt caggagctaa ggaagctaaa    1800
   atggagaaaa aaatcactgg atataccacc gttgatatat cccaatggca tcgtaaagaa 1860
   cattttgagg catttcagtc agttgctcaa tgtacctata accagaccgt tcagctggat 1920
   attacggcct ttttaaagac cgtaaagaaa aataagcaca agttttatcc ggcctttatt 1980
   cacattcttg cccgcctgat gaatgctcat ccggagtttc gtatggcaat gaaagacggt 2040
   gagctggtga tatgggatag tgttcaccct tgttacaccg ttttccatga gcaaactgaa 2100
   acgttttcat cgctctggag tgaataccac gacgatttcc ggcagtttct acacatatat 2160
   tcgcaagatg tggcgtgtta cggtgaaaac ctggcctatt tccctaaagg gtttattgag 2220
   aatatgtttt tcgtctcagc caatccctgg gtgagtttca ccagttttga tttaaacgtg 2280
   gccaatatgg acaacttctt cgcccccgtt ttcaccatgg gcaaatatta tacgcaaggc 2340
   gacaaggtgc tgatgccgct ggcgattcag gttcatcatg ccgtttgtga tggcttccat 2400
   gtcggcagaa tgcttaatga attacaacag tactgcgatg agtggcaggg cggggcgtaa 2460
   tttttttaag gcgacaccat cgaatggcgc aaaacctttc gcggtatggc atgatagcgc 2520
   ccggaagaga gtcaattcag ggtggtgaat gtgaaaccag taacgttata cgatgtcgca 2580
   gagtatgccg gtgtctctta tcagaccgtt tcccgcgtgg tgaaccaggc cagccacgtt 2640
   tctgcgaaaa cgcgggaaaa agtggaagcg gcgatggcgg agctgaatta cattcccaac 2700
   cgcgtggcac aacaactggc gggcaaacag tcgttgctga ttggcgttgc cacctccagt 2760
   ctggccctgc acgcgccgtc gcaaattgtc gcggcgatta aatctcgcgc cgatcaactg 2820
   ggtgccagcg tggtggtgtc gatggtagaa cgaagcggcg tcgaagcctg taaagcggcg 2880
   gtgcacaatc ttctcgcgca acgcgtcagt gggctgatca ttaactatcc gctggatgac 2940
   caggatgcca ttgctgtgga agctgcctgc actaatgttc cggcgttatt tcttgatgtc 3000
   tctgaccaga cacccatcaa cagtattatt ttctcccatg aagacggtac gcgactgggc 3060
   gtggagcatc tggtcgcatt gggtcaccag caaatcgcgc tgttagcggg cccattaagt 3120
   tctgtctcgg cgcgtctgcg tctggctggc tggcataaat atctcactcg caatcaaatt 3180
   cagccgatag cggaacggga aggcgactgg agtgccatgt ccggttttca acaaaccatg 3240
   caaatgctga atgagggcat cgttcccact gcgatgctgg ttgccaacga tcagatggcg 3300
   ctgggcgcaa tgcgcgccat taccgagtcc gggctgcgcg ttggtgcgga tatctcggta 3360
   gtgggatacg acgataccga agacagctca tgttatatcc cgccgttaac caccatcaaa 3420
   caggattttc gcctgctggg gcaaaccagc gtggaccgct tgctgcaact ctctcagggc 3480
   caggcggtga agggcaatca gctgttgccc gtctcactgg tgaaaagaaa aaccaccctg 3540
   gcgcccaata cgcaaaccgc ctctccccgc gcgttggccg attcattaat gcagctggca 3600
   cgacaggttt cccgactgga aagcgggcag tgagtggata accgtattac cgcctttgag 3660
   tgagctgata ccgggaattc tcactcatta ggcaccccag gctttacact ttatgcttcc 3720
   ggctcgtata atgtgtggaa ttgtgagcgg ataacaattt cacacaggaa agcttcatat 3780
   gaataaggcc tacagtatca tttggagcca ctccagacag gcctggattg tggcctcaga 3840
   gttagccaga ggacatggtt ttgtccttgc aaaaaataca ctgctggtat tggcggttgt 3900
   ttccacaatc ggaaatgcat ttgcagtcga ccaccatcac catcaccatc tggaagcgct 3960
   gttccagggt ccgggtaccg gtaacaagta tagagttaga aaaaacgtat tacatcttac 4020
   cgacacggaa aaaagagatt ttgttcgtac cgtgctaata ctaaaggaaa aagggatata 4080
   tgaccgctat atagcctggc atggtgcagc aggtaaattt catactcctc cgggcagcga 4140
   tcgaaatgca gcacatatga gttctgcttt tttaccgtgg catcgtgaat accttttacg 4200
   attcgaacgt gaccttcagt caatcaatcc agaagtaacc cttccttatt gggaatggga 4260
   aacggacgca cagatgcagg atccctcaca atcacaaatt tggagtgcag attttatggg 4320
   aggaaacgga aatcccataa aagattttat cgtcgatacc gggccatttg cagctgggcg 4380
   ctggacgacg atcgatgaac aaggaaatcc ttccggaggg ctaaaacgta attttggagc 4440
   aacgaaagag gcacctacac tccctactcg agatgatgtc ctcaatgctt taaaaataac 4500
   tcagtatgat acgccgcctt gggatatgac cagccaaaac agctttcgta atcagcttga 4560
   aggatttatt aacgggccac agcttcacaa tcgcgtacac cgttgggttg gcggacagat 4620
   gggcgttgtg cctactgctc cgaatgatcc tgtcttcttt ttacaccacg caaatgtgga 4680
   tcgtatttgg gctgtatggc aaattattca tcgtaatcaa aactatcagc cgatgaaaaa 4740
   cgggccattt ggtcaaaact ttagagatcc gatgtaccct tggaatacaa cccctgaaga 4800
   cgttatgaac catcgaaagc ttgggtacgt atacgatata gaattaagaa aatcaaaacg 4860
   ttcctcagag ctcgaaaacc tgtacttcca gggtgaacag aaactgatta gcgaagaaga 4920
   tctgtctaga gtgaataaca atggaagcat tgtcattaat aacagcatta taaacgggaa 4980
   tattacgaat gatgctgact taagttttgg tacagcaaag ctgctctctg ctacagtgaa 5040
   tggtagtctt gttaataaca aaaatatcat tcttaatcct acaaaagaaa gtgcggccgc 5100
   tataggtaat actcttaccg tgtcaaatta tactgggaca ccgggaagtg ttatttctct 5160
   tggtggtgtg cttgaaggag ataattcact tacggaccgt ctggtggtga aaggtaatac 5220
   ctctggtcaa agtgacatcg tttatgtcaa tgaagatggc agtggtggtc agacgagaga 5280
   tggtattaat attatttctg tagagggaaa ttctgatgca gaattctctc tgaagaaccg 5340
   cgtagttgcc ggagcttatg attacacact gcagaaagga aacgagagtg ggacagataa 5400
   taagggatgg tatttaacca gtcatcttcc cacatctgat acccggcaat acagaccgga 5460
   gaacggaagt tatgctacca atatggcact ggctaactca ctgttcctca tggatttgaa 5520
   tgagcgtaag caattcaggg ccatgagtga taatacacag cctgagtctg catccgtgtg 5580
   gatgaagatc actggaggaa taagctctgg taagctgaat gacgggcaaa ataaaacaac 5640
   aaccaatcag tttatcaatc agctcggggg ggatatttat aaattccatg ctgaacaact 5700
   gggtgatttt accttaggga ttatgggagg atacgcgaat gcaaaaggta aaacgataaa 5760
   ttacacgagc aacaaagctg ccagaaacac actggatggt tattctgtcg gggtatacgg 5820
   tacgtggtat cagaatgggg aaaatgcaac agggctcttt gctgaaactt ggatgcaata 5880
   taactggttt aatgcatcag tgaaaggtga cggactggaa gaagaaaaat ataatctgaa 5940
   tggtttaacc gcttctgcag gtgggggata taacctgaat gtgcacacat ggacatcacc 6000
   tgaaggaata acaggtgaat tctggttaca gcctcatttg caggctgtct ggatgggggt 6060
   tacaccggat acacatcagg aggataacgg aacggtggtg cagggagcag ggaaaaataa 6120
   tattcagaca aaagcaggta ttcgtgcatc ctggaaggtg aaaagcaccc tggataagga 6180
   taccgggcgg aggttccgtc cgtatataga ggcaaactgg atccataaca ctcatgaatt 6240
   tggtgttaaa atgagtgatg acagccagtt gttgtcaggt agccgaaatc agggagagat 6300
   aaagacaggt attgaagggg tgattactca aaacttgtca gtgaatggcg gagtcgcata 6360
   tcaggcagga ggtcacggga gcaatgccat ctccggagca ctggggataa aatacagctt 6420
   ctgataatga tcc 6433
<210> 5
   <211> 2649
   <212> DNA
   <213> Artificial sequence
<220>
   <223> Nucleic acid sequence encoding protein expressed from the vector pAIDA1-Tyr1
<400> 5
   atgaataagg cctacagtat catttggagc cactccagac aggcctggat tgtggcctca      60
   gagttagcca gaggacatgg ttttgtcctt gcaaaaaata cactgctggt attggcggtt     120
   gtttccacaa tcggaaatgc atttgcagtc gaccaccatc accatcacca tctggaagcg     180
   ctgttccagg gtccgggtac cggtaacaag tatagagtta gaaaaaacgt attacatctt     240
   accgacacgg aaaaaagaga ttttgttcgt accgtgctaa tactaaagga aaaagggata     300
   tatgaccgct atatagcctg gcatggtgca gcaggtaaat ttcatactcc tccgggcagc     360
   gatcgaaatg cagcacatat gagttctgct tttttaccgt ggcatcgtga atacctttta      420
   cgattcgaac gtgaccttca gtcaatcaat ccagaagtaa cccttcctta ttgggaatgg     480
   gaaacggacg cacagatgca ggatccctca caatcacaaa tttggagtgc agattttatg     540
   ggaggaaacg gaaatcccat aaaagatttt atcgtcgata ccgggccatt tgcagctggg     600
   cgctggacga cgatcgatga acaaggaaat ccttccggag ggctaaaacg taattttgga     660
   gcaacgaaag aggcacctac actccctact cgagatgatg tcctcaatgc tttaaaaata 720
   actcagtatg atacgccgcc ttgggatatg accagccaaa acagctttcg taatcagctt 780
   gaaggattta ttaacgggcc acagcttcac aatcgcgtac accgttgggt tggcggacag 840
   atgggcgttg tgcctactgc tccgaatgat cctgtcttct ttttacacca cgcaaatgtg 900
   gatcgtattt gggctgtatg gcaaattatt catcgtaatc aaaactatca gccgatgaaa 960
   aacgggccat ttggtcaaaa ctttagagat ccgatgtacc cttggaatac aacccctgaa 1020
   gacgttatga accatcgaaa gcttgggtac gtatacgata tagaattaag aaaatcaaaa 1080
   cgttcctcag agctcgaaaa cctgtacttc cagggtgaac agaaactgat tagcgaagaa 1140
   gatctgtcta gagtgaataa caatggaagc attgtcatta ataacagcat tataaacggg 1200
   aatattacga atgatgctga cttaagtttt ggtacagcaa agctgctctc tgctacagtg 1260
   aatggtagtc ttgttaataa caaaaatatc attcttaatc ctacaaaaga aagtgcggcc 1320
   gctataggta atactcttac cgtgtcaaat tatactggga caccgggaag tgttatttct 1380
   cttggtggtg tgcttgaagg agataattca cttacggacc gtctggtggt gaaaggtaat 1440
   acctctggtc aaagtgacat cgtttatgtc aatgaagatg gcagtggtgg tcagacgaga 1500
   gatggtatta atattatttc tgtagaggga aattctgatg cagaattctc tctgaagaac 1560
   cgcgtagttg ccggagctta tgattacaca ctgcagaaag gaaacgagag tgggacagat 1620
   aataagggat ggtatttaac cagtcatctt cccacatctg atacccggca atacagaccg 1680
   gagaacggaa gttatgctac caatatggca ctggctaact cactgttcct catggatttg 1740
   aatgagcgta agcaattcag ggccatgagt gataatacac agcctgagtc tgcatccgtg 1800
   tggatgaaga tcactggagg aataagctct ggtaagctga atgacgggca aaataaaaca 1860
   acaaccaatc agtttatcaa tcagctcggg ggggatattt ataaattcca tgctgaacaa 1920
   ctgggtgatt ttaccttagg gattatggga ggatacgcga atgcaaaagg taaaacgata 1980
   aattacacga gcaacaaagc tgccagaaac acactggatg gttattctgt cggggtatac 2040
   ggtacgtggt atcagaatgg ggaaaatgca acagggctct ttgctgaaac ttggatgcaa 2100
   tataactggt ttaatgcatc agtgaaaggt gacggactgg aagaagaaaa atataatctg     2160
   aatggtttaa ccgcttctgc aggtggggga tataacctga atgtgcacac atggacatca     2220
   cctgaaggaa taacaggtga attctggtta cagcctcatt tgcaggctgt ctggatgggg 2280
   gttacaccgg atacacatca ggaggataac ggaacggtgg tgcagggagc agggaaaaat 2340
   aatattcaga caaaagcagg tattcgtgca tcctggaagg tgaaaagcac cctggataag 2400
   gataccgggc ggaggttccg tccgtatata gaggcaaact ggatccataa cactcatgaa 2460
   tttggtgtta aaatgagtga tgacagccag ttgttgtcag gtagccgaaa tcagggagag 2520
   ataaagacag gtattgaagg ggtgattact caaaacttgt cagtgaatgg cggagtcgca 2580
   tatcaggcag gaggtcacgg gagcaatgcc atctccggag cactggggat aaaatacagc 2640
   ttctgataa 2649
<210> 6
   <211> 440
   <212> PRT
   <213> Artificial sequence
<220>
   <223> Amino acid sequence protein AIDAC
<400> 6
   Ile Gly Asn Thr Leu Thr Val Ser Asn Tyr Thr Gly Thr Pro Gly Ser
   1               5                   10                  15
   Val Ile Ser Leu Gly Gly Val Leu Glu Gly Asp Asn Ser Leu Thr Asp
               20                  25                  30
   Arg Leu Val Val Lys Gly Asn Thr Ser Gly Gln Ser Asp Ile Val Tyr
           35                  40                  45
   Val Asn Glu Asp Gly Ser Gly Gly Gln Thr Arg Asp Gly Ile Asn Ile
       50                  55                  60
   Ile Ser Val Glu Gly Asn Ser Asp Ala Glu Phe Ser Leu Lys Asn Arg
   65                  70                  75                  80
   Val Val Ala Gly Ala Tyr Asp Tyr Thr Leu Gln Lys Gly Asn Glu Ser
                   85                  90                  95
   Gly Thr Asp Asn Lys Gly Trp Tyr Leu Thr Ser His Leu Pro Thr Ser
               100                 105                 110
   Asp Thr Arg Gln Tyr Arg Pro Glu Asn Gly Ser Tyr Ala Thr Asn Met
           115                 120                 125
   Ala Leu Ala Asn Ser Leu Phe Leu Met Asp Leu Asn Glu Arg Lys Gln
       130                 135                 140
   Phe Arg Ala Met Ser Asp Asn Thr Gln Pro Glu Ser Ala Ser Val Trp
   145                 150                 155                 160
   Met Lys Ile Thr Gly Gly Ile Ser Ser Gly Lys Leu Asn Asp Gly Gln
                   165                 170                 175
   Asn Lys Thr Thr Thr Asn Gln Phe Ile Asn Gln Leu Gly Gly Asp Ile
               180                 185                 190
   Tyr Lys Phe His Ala Glu Gln Leu Gly Asp Phe Thr Leu Gly Ile Met
           195                 200                 205
   Gly Gly Tyr Ala Asn Ala Lys Gly Lys Thr Ile Asn Tyr Thr Ser Asn
       210                 215                 220
   Lys Ala Ala Arg Asn Thr Leu Asp Gly Tyr Ser Val Gly Val Tyr Gly
   225                 230                 235                 240
   Thr Trp Tyr Gln Asn Gly Glu Asn Ala Thr Gly Leu Phe Ala Glu Thr
                   245                 250                 255
   Trp Met Gln Tyr Asn Trp Phe Asn Ala Ser Val Lys Gly Asp Gly Leu
               260                 265                 270
   Glu Glu Glu Lys Tyr Asn Leu Asn Gly Leu Thr Ala Ser Ala Gly Gly
           275                 280                 285
   Gly Tyr Asn Leu Asn Val His Thr Trp Thr Ser Pro Glu Gly Ile Thr
       290                 295                 300
   Gly Glu Phe Trp Leu Gln Pro His Leu Gln Ala Val Trp Met Gly Val
   305                 310                 315                 320
   Thr Pro Asp Thr His Gln Glu Asp Asn Gly Thr Val Val Gln Gly Ala
                   325                 330                 335
   Gly Lys Asn Asn Ile Gln Thr Lys Ala Gly Ile Arg Ala Ser Trp Lys
               340                 345                 350
   Val Lys Ser Thr Leu Asp Lys Asp Thr Gly Arg Arg Phe Arg Pro Tyr
           355                 360                 365
   Ile Glu Ala Asn Trp Ile His Asn Thr His Glu Phe Gly Val Lys Met
       370                 375                 380
   Ser Asp Asp Ser Gln Leu Leu Ser Gly Ser Arg Asn Gln Gly Glu Ile
   385                 390                 395                 400
   Lys Thr Gly Ile Glu Gly Val Ile Thr Gln Asn Leu Ser Val Asn Gly
                   405                 410                 415
   Gly Val Ala Tyr Gln Ala Gly Gly His Gly Ser Asn Ala Ile Ser Gly
               420                 425                 430
   Ala Leu Gly Ile Lys Tyr Ser Phe
           435                 440
<210> 7
   <211> 35
   <212> DNA
   <213> Artificial sequence
<220>
   <223> primer
<400> 7
   agcggtaccg gtaacaagta tagagttaga aaaaa 35
<210> 8
   <211> 32
   <212> DNA
   <213> Artificial sequence
<220>
   <223> Primer
<400> 8
   aaagagctct gaggaacgtt ttgattttct ta 32
<210> 9
   <211> 27
   <212> DNA
   <213> Artificial sequence
<220>
   <223> Primer
<400> 9
   aaaggtaccg cgtggctggt cggcaag 27
<210> 10
   <211> 31
   <212> DNA
   <213> Artificial sequence
<220>
   <223> Primer
<400> 10
   agcgagctcg gcggacacta tggctatttc t 31
<210> 11
   <211> 27
   <212> DNA
   <213> Artificial sequence
<220>
   <223> Primer
<400> 11
   aaaggtacct caaatccaag ggagagt 27
<210> 12
   <211> 27
   <212> DNA
   <213> Artificial sequence
<220>
   <223> Primer
<400> 12
   aaagagctcg accttgaagt aggtccg 27
<210> 13
   <211> 41
   <212> DNA
   <213> Artificial sequence
<220>
   <223> Primer
<400> 13
   gaaaagcttc atatgggtaa caagtataga gttagaaaaa a 41
<210> 14
   <211> 37
   <212> DNA
   <213> Artificial sequence
<220>
   <223> Primer
<400> 14
   aaaggcgcgc cttatgagga acgttttgat tttctta 37
<210> 15
   <211> 894
   <212> DNA
   <213> Artificial sequence
<220>
   <223> Tyr1 (coding sequence)
<400> 15
   atgggtaaca agtatagagt tagaaaaaac gtattacatc ttaccgacac ggaaaaaaga      60
   gattttgttc gtaccgtgct aatactaaag gaaaaaggga tatatgaccg ctatatagcc     120
   tggcatggtg cagcaggtaa atttcatact cctccgggca gcgatcgaaa tgcagcacat     180
   atgagttctg cttttttacc gtggcatcgt gaataccttt tacgattcga acgtgacctt    240
   cagtcaatca atccagaagt aacccttcct tattgggaat gggaaacgga cgcacagatg     300
   caggatccct cacaatcaca aatttggagt gcagatttta tgggaggaaa cggaaatccc     360
   ataaaagatt ttatcgtcga taccgggcca tttgcagctg ggcgctggac gacgatcgat     420
   gaacaaggaa atccttccgg agggctaaaa cgtaattttg gagcaacgaa agaggcacct     480
   acactcccta ctcgagatga tgtcctcaat gctttaaaaa taactcagta tgatacgccg     540
   ccttgggata tgaccagcca aaacagcttt cgtaatcagc ttgaaggatt tattaacggg     600
   ccacagcttc acaatcgcgt acaccgttgg gttggcggac agatgggcgt tgtgcctact      660
   gctccgaatg atcctgtctt ctttttacac cacgcaaatg tggatcgtat ttgggctgta      720
   tggcaaatta ttcatcgtaa tcaaaactat cagccgatga aaaacgggcc atttggtcaa      780
   aactttagag atccgatgta cccttggaat acaacccctg aagacgttat gaaccatcga      840
   aagcttgggt acgtatacga tatagaatta agaaaatcaa aacgttcctc ataa            894